(19) **Europäisches Patentamt / European Patent Office / Office européen des brevets**

(11) **EP 2 763 690 B1**

(12) # EUROPEAN PATENT SPECIFICATION

(45) Date of publication and mention of the grant of the patent:
**25.11.2015 Bulletin 2015/48**

(21) Application number: **12766695.6**

(22) Date of filing: **02.10.2012**

(51) Int Cl.:
*A61K 38/26* (2006.01)       *A61P 1/18* (2006.01)
*A61P 35/00* (2006.01)

(86) International application number:
**PCT/EP2012/069485**

(87) International publication number:
**WO 2013/050379 (11.04.2013 Gazette 2013/15)**

(54) **LIXISENATIDE FOR USE IN THE TREATMENT OF STENOSIS OR/AND OBSTRUCTION IN THE PANCREATIC DUCT SYSTEM**

LIXISENATIDE ZUR BEHANDLUNG VON STENOSEN ODER/UND OBSTRUKTIONEN IM PANKREASGANGSYSTEM

LIXISENATIDE POUR L'UTILISATION DANS LE TRAITEMENT DE LA STÉNOSE ET/OU DE L'OBSTRUCTION DANS LE SYSTÈME DES CONDUITS PANCRÉATIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.10.2011 EP 11183867**

(43) Date of publication of application:
**13.08.2014 Bulletin 2014/33**

(73) Proprietor: **Sanofi-Aventis Deutschland GmbH 65929 Frankfurt am Main (DE)**

(72) Inventors:
• **STECHL, Jens**
  **65929 Frankfurt am Main (DE)**
• **NOWOTNY, Irene**
  **65929 Frankfurt am Main (DE)**
• **PFEIFFER, Claudia**
  **65929 Frankfurt am Main (DE)**
• **PINQUIER, Jean-Louis**
  **F-92140 Clamart (FR)**
• **MSIHID, Jerome**
  **F-75008 Paris (FR)**

(74) Representative: **Weickmann & Weickmann Postfach 860 820 81635 München (DE)**

(56) References cited:
**WO-A1-98/05351      WO-A2-2007/028394
US-A- 5 187 177**

• **HELLSTROM P M ET AL: "T1388 GTP-010 As a Therapeutic Tool in IBS Pain Relief: Prospective, Randomized, Placebo-Controlled Study of a GLP-1 Analog", GASTROENTEROLOGY, ELSEVIER, PHILADELPHIA, PA, vol. 134, no. 4, 1 April 2008 (2008-04-01) , pages A-544, XP023434339, ISSN: 0016-5085, DOI: 10.1016/S0016-5085(08)62542-1 [retrieved on 2008-04-01]**
• **CHRISTENSEN M ET AL: "Lixisenatide, a novel GLP-1 receptor agonist for the treatment of type 2 diabetes mellitus", I DRUGS: THE INVESTIGATIONAL DRUGS JOURNAL, CURRENT DRUGS LTD, GB, vol. 12, no. 8, 1 August 2009 (2009-08-01) , pages 503-513, XP002572656, ISSN: 2040-3410**

**Description**

The present invention relates to a pharmaceutical composition for use in the treatment of a disease or condition, wherein said disease or condition is associated with stenosis or/and obstruction located in the pancreatic duct system, said composition comprising lixisenatide or/and a pharmaceutically acceptable salt thereof and optionally a pharmaceutically acceptable carrier, diluent or/and auxiliary substance.

The pancreas is an exocrine and endocrine gland. The endocrine pancreas (Langerhans islets) produces insulin which is secreted into the blood stream. The exocrine pancreas produces pancreatic juice containing digestive enzymes, for example proteases, lipases, and nucleases, and bicarbonate. The enzymes can be provided as proenzymes. The pancreatic duct system directs the pancreatic juice into the duodenum.

Anatomically the pancreas body (corpus pancreatis) is distinguished from the head (caput pancreatis) and the tail (cauda pancreatis).

The pancreatic duct system includes the major pancreatic duct (also termed ductus pancreaticus major, main pancreatic duct or duct of Wirsung). The major pancreatic duct leads to the ductus hepatopancreaticus. The ductus heptopancreaticus is formed by junction of the common bile duct and the major pancreatic duct, leading to the papilla of Vater (also termed papilla vateri or papilla duodeni major) located in the duodenum wall. The papilla of Vater includes the sphincter of Oddi. The sphincter of Oddi consists of smooth muscle fibers surrounding a variable length of the ductus hepatopancreaticus.

In the pancreatic duct system, some non-pathologic variants exist. For example, the ductus heptopancreaticus can be enlarged to form the ampulla hepatopancreatica (also termed ampulla of Vater). In another variant, the ductus hepatopancreaticus may be short or even absent. This means that the common bile duct (also termed ductus choledochus) and the major pancreatic duct lead independently into the duodenum.

In yet another variant, an accessory pancreatic duct (ductus pancreaticus accessorius, duct of Santorini) may be present, which leads into the duodenum on the minor duodenal papilla (also termed papilla duodeni minor). The accessory pancreatic duct by-passes the ductus hepatopancreaticus and the papilla of Vater.

A common congenital anomaly is pancreas divisium (complete pancreas divisum or incomplete pancreas division) which results from abnormal fusion between the ventral and dorsal pancreatic ducts during fetal development. In complete pancreas divisum, a completely separate pancreatic duct system exists, leading to the papilla of Vater and the minor duodenal papilla. Incomplete pancreas divisum is a pancreatic anomaly with inadequate communication between the ventral and dorsal pancreatic duct. Pancreaticobiliary malfunction is a congenital anomaly defined as the union of the pancreatic ducts and the biliary ducts outside the duodenal wall (Kamisawa et al., J. Anat. 2008, 212(2):125-34).

The pancreatic duct system and the biliary system are histologically related. The pancreatic duct system and the biliary tract (including ductus hepaticus, gall bladder, ductus cysticus, ductus choledochus) have a similar histological structure: the walls comprise smooth muscle, and the inner surface is covered by columnar epithelium.

The contraction of the smooth muscle in the biliary tract (in particular in the gall bladder) and in the pancreatic duct system can be induced by cholecystokinin (CCK). CCK is produced in the mucosa of the small intestine. CCK reaches the gall bladder via the circulation. Release of CCK can be induced by a fatty meal. By contraction of the gall bladder, bile is released into the duodenum, where it helps emulsifying fats. Another function of CCK is the induction of release of digestive enzymes in the stomach and in the pancreas.

Stenosis or/and obstruction in the pancreatic duct system can lead to an impaired flow of pancreatic juice, resulting in a condition associated with pain. Stenotic processes or/and obstruction within the pancreatic duct system, for example by inwards growth of surrounding tissue (by cancer or/and inflammatory processes), may cause an increase of intraduct volume by accumulation of pancreatic juice, combined with dilation of the duct wall. The pancreatic juice contains dissolved pancreatic enzymes (e.g. amylase and lipase), which, when accumulated in the pancreatic duct system, will become autoaggressive, resulting in a perpetuation of the inflammatory process in the pancreatic duct system. Furthermore, the increase of volume, which may be combined with dilation of duct wall, may result in severe acute or chronic pain. Treatment of such pain includes treatment with opioid analgesics or by surgical removal of the stenosis or/and obstruction. Such surgery may be a "radical" surgery, i.e. complete removal of the affected tissue.

Obstruction can be caused by concrements, for example calcium bicarbonate concrements. These concrements are also termed pancreatic stones. Such concrements include pancreoliths. A synonyme for "pancreolith" is "pancreatic calculus". The presence of concrements in the pancreases is called pancreolithiasis. Concrements in the pancreas can be formed for example during pancreatitis. Concrements can be originated by a dysbalance of enzyme concentration and inflammatory cells, resulting in a precipitation. Even more inflammatory reaction can be induced, and inflammation and concrement formation perpetuates. See, for example, Marvin Sleisenger (ed.) Gastrointestinal disease: pathophysiology, diagnosis, management, Saunders, Philadelphia, 1973, 1983, 1993.

A stenosis of the pancreatic duct system can also be originated by cancer, for example exocrine pancreatic cancers or/and endocrine pancreatic cancers. By growth, an exocrine pancreatic cancer or/and an endocrine pancreatic cancer can cause a stenosis of the pancreatic duct system. Examples of pancreatic cancers include, but are not limited to pancreatic cystadenoma, pancreatic cystadenocarcinoma, pancreatic adenoacanthoma, and secretory tumors, such as multiple endocrine neoplasia, insulinoma, glucagonoma, and somatostatinoma. See, for example, Marvin Sleisenger (ed.) Gastrointestinal disease: pathophysiology, diagnosis, management, Saunders, Philadelphia, 1973, 1983, 1993.

Glucagon-like peptide 1 (GLP-1) is an endocrine hormone which increases the insulin response following oral intake of glucose or fat. GLP-1 generally regulates the concentrations of glucagons, slows down gastric emptying, stimulates the biosynthesis of (pro-)insulin, increases the sensitivity toward insulin, and stimulates the insulin-independent biosynthesis of glycogen (Holst (1999), Curr. Med. Chem 6: 1005, Nauck et al. (1997) Exp Clin Endocrinol Diabetes 105: 187, Lopez-Delgado et al. (1998) Endocrinology 139:2811).

Human GLP-1 has 37 amino acid residues (Heinrich et al., Endocrinol. 115:2176. (1984), Uttenthal et al., J Clin Endocrinol Metabol (1985) 61:472). Active fragments of GLP-1 include GLP-1 (7-36) amide and GLP-1 (7-37).

Exendins are a group of peptides which are able to lower blood glucose concentrations. Exendins have a certain similarity in sequence to GLP-1 (7-36) (53%, Goke et al. J. Biol Chem 268, 19650-55). Exendin-3 and exendin-4 stimulate an increase in cellular cAMP production in acinar cells of the guinea pig pancreas by interaction with exendin receptors (Raufman, 1996, Reg. Peptides 61: 1-18). In contrast to exendin-4, exendin-3 produces an increase in amylase release in acinar cells of the pancreas.

Exendin-3, exendin-4, and exendin agonists have been proposed for the treatment of diabetes mellitus and the prevention of hyperglycemia; they reduce gastric motility and gastric emptying (US 5,424,286 and WO98/05351).

Exendin analogues may be characterized by amino acid replacements and/or C-terminal truncation of the natural exendin-4 sequence. Exendin analogues of this kind are described in WO 99/07404, WO 99/25727, WO 99/25728. Exendin-4 analogues include lixisenatide (also termed AVE0010, desPro$^{36}$-exendin-4-Lys$_6$-NH$_2$ or H-desPro$^{36}$-exendin-4-Lys$_6$-NH$_2$).

WO 2007/028394 discloses the use of a GLP-1 molecule for the treatment of biliary dyskinesia and/or biliary pain/discomfort. According to WO 2007/028394, biliary dyskinesia may be an increased motility of an area of the biliary tract, or a decreased motility of an area of the biliary tract. The GLP-1 molecule is considered as prokinetic agent. According to WO 2007/028394, the GLP-1 molecule may be administered in combination with one or more other excitatory factor(s) capable of inducing bile flow and/or treating biliary tract motility disorders. Alternatively, the GLP-1 molecule may be administered in combination with one or more inhibitory factor(s) capable of reducing bile flow. No experimental evidence is presented in WO 2007/028394 that a GLP-1 molecule would affect gallbladder physiology.

GLP-1 is not known to have any effect on the pancreatic duct system.

Gallbladder motility can be expressed by gallbladder ejection fraction and can be assessed by cholescintigraphy. Cholescintigraphy is a nuclear imaging procedure to evaluate the function of the gallbladder. For example, a derivative of iminodiacetic acid labelled with $^{99m}$Technetium is injected i.v., then allowed to circulate to the liver, where it is excreted into the biliary system and stored by the gallbladder. Cholescintigraphy is used for diagnosis of gallbladder dyskinesia, which involves gallbladder dysfunction. Cases with a gall bladder ejection fraction (GBEF) below 40% are considered to be indicated for cholecystectomy (Behar J. et al., Gastroenterology 2006 130:1498-1509).

Relaxation and contraction of the gallbladder is mediated by smooth muscle cells and represent the morphological correlate of the dynamic measurements via the $^{99m}$technetium cholescintigraphy method.

The example of the present invention refers to a randomized, double-blind, placebo-controlled, two-sequence, two-treatment cross-over study assessing the effect of a single subcutaneous injection of 20 μg lixisenatide on gallbladder motility in healthy male and female subjects. Gallbladder motility has been analysed by cholescintigraphy. Cholecystokinin (CCK-8) has been administrated 60 min after administration of a single dose of placebo or 20 μg lixisenatide and followed immediately by a single dose of $^{99m}$Tc mebrofenin (a $^{99m}$Tc-labeled iminodiacetic acid derivative). The procedure can be summarized as follows. By placing a radiation-sensitive camera over the subject's abdomen, a "picture" of the liver, bile ducts, and gallbladder can be obtained that corresponds to where the radioactive bile has migrated. After injection of lixisenatide or placebo and $^{99m}$Tc injection, hepatic frame images were obtained at 1 frame/min for 60 min. After gallbladder visualization at 60 min, 0.02 μg/kg CCK-8 was administered via constant infusion pump for 60 min. Image acquisition was continued for at least an additional 60 min following CCK-8 infusion.

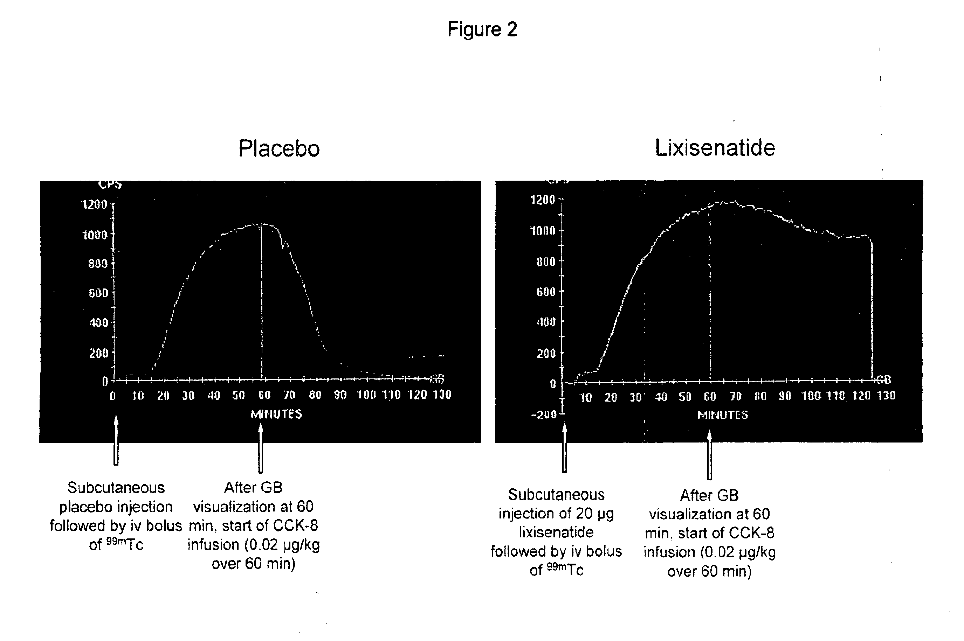

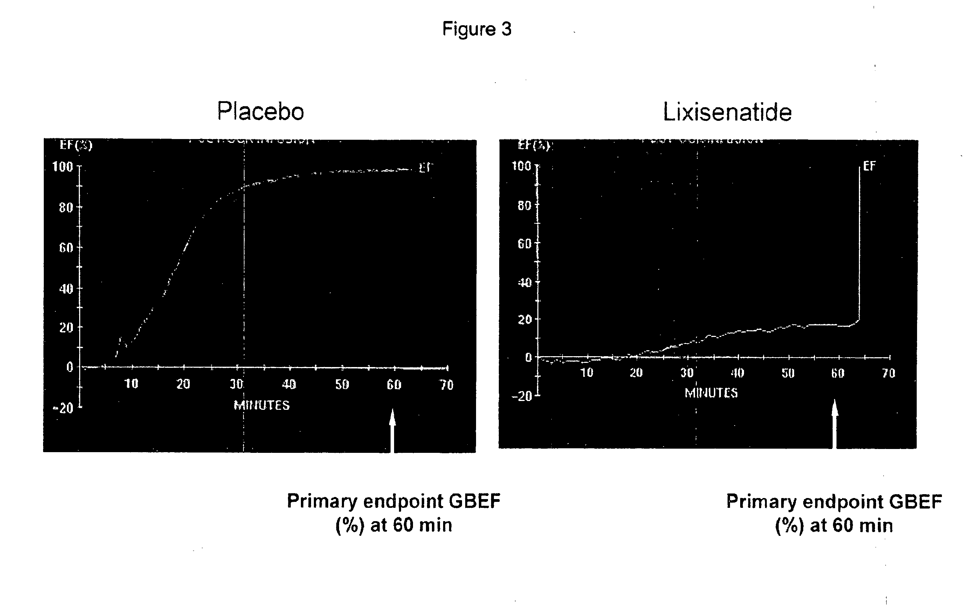

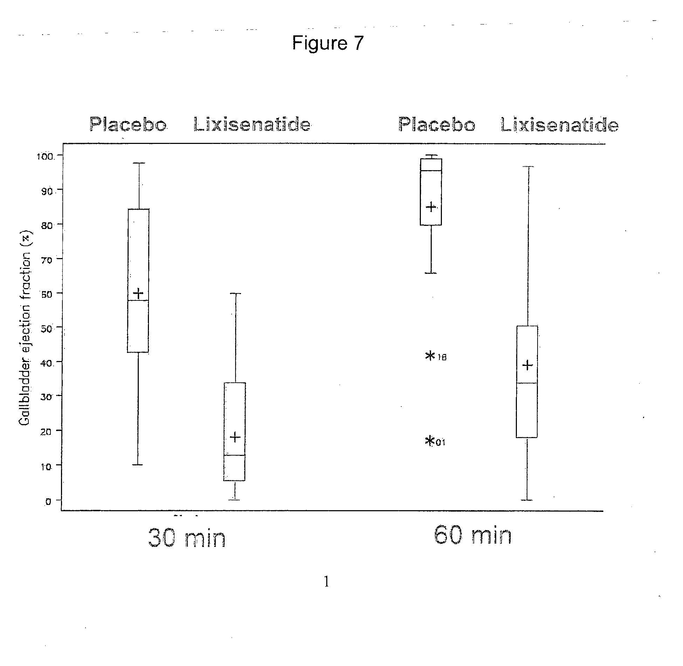

In the placebo group, CCK-8 induced a decline of counts recorded from the gallbladder, indicating a release of bile from the gallbladder via bile ducts into the duodenum. Surprisingly, by subcutaneous administration of a single dose of 20 μg lixisenatide in healthy subjects, this effect of CCK-8 is largely reduced (see exemplary filling and emptying curves in Figure 2). The amount of $^{99m}$Tc remains high in the gallbladder, indicating that only a small fraction of bile has been released. The effect has been quantified as follows. In the placebo group, CCK-8 induces an increase of the gallbladder ejection fraction (GBEF, or ejection fraction EF, i.e. the volume fraction of bile ejected from the gallbladder,) up to about 85%, as expected from the physiological action of CCK. In the lixisenatide group, the ejection fraction remains below 40% (see exemplary GBEF curve in Figure 3, averages given in Figure 7 and Table 9) after CCK-8 administration. Under lixisenatide, 13/24 (54%) subjects had a GBEF at 60 min of below 40% compared to 1/24 (4%) under placebo. The subcutaneous administration of a single dose of 20 μg lixisenatide in healthy subjects significantly reduced gallbladder emptying expressed as gallbladder ejection fraction (GBEF) in response to CCK-8 compared to placebo at 60 min by 45.8%.

The origin of the lixisenatide effect upon GBEF, as described herein, is not known. Without wishing to be bound by theory, reduction of CCK-induced gallbladder emptying under lixisenatide can be explained by lixisenatide-induced smooth muscle relaxation of the gallbladder. Lixisenatide thus provides a spasmolytic effect in the gallbladder. As the pancreatic duct system and the biliary tract share significant similarities with respect to smooth muscle cells in the ducts, one can conclude that lixisenatide will exhibit a similar smooth muscle relaxation effect in the pancreatic duct system as in the biliary system. Based upon the lixisenatide effect upon GBEF disclosed herein, it is expected that the smooth muscle cells in the pancreatic duct system may become relaxed by administration of lixisenatide or a GLP-1 agonist. Thus, GLP-1 agonists including Lixisenatide may provide a spasmolytic or antispasmodic effect in the pancreatic duct system.

A stenosis or/and obstruction in the biliary tract or in the pancreatic duct system may result in a spasm, which often is painful. The spasmolytic effects of GLP-1 agonists propose the therapeutic use of GLP-1 agonists in the treatment of diseases associated with stenotic or obstructive processes. As the pancreatic duct system and the biliary system are anatomically and histologically related, GLP-1 agonists, such as lixisenatide, can exhibit a therapeutic effect on stenotic or obstructive processes not only in the biliary tract, but also in the pancreatic duct system or/and circumventing pancreatic tissue. Relaxation of the smooth muscle results in an increase of duct lumen, thus improving the flow of bile or/and pancreatic juice if a stenosis or.an obstruction is present. By the spasmolytic activity, the GLP-1 agonists becomes suitable for the treatment of pain associated with stenosis or/and obstruction in the biliary system or/and the pancreatic duct system.

Described herein is a pharmaceutical composition for use in the treatment of a disease or condition, wherein said disease or condition is associated with stenosis or/and obstruction located in the pancreatic duct system, said composition comprising at least one GLP-1 agonist and optionally a pharmaceutically acceptable carrier, diluent or/and auxiliary substance.

A first aspect of the present invention refers to a pharmaceutical composition for use in the treatment of a disease or condition, wherein said disease or condition is associated with stenosis or/and obstruction located in the pancreatic duct system, said composition comprising lixisenatide or/and a pharmaceutically acceptable salt thereof and optionally a pharmaceutically acceptable carrier, diluent or/and auxiliary substance. The stenosis or/and obstruction may also be located in the circumventing pancreatic tissue.

In the present invention, "pancreatic duct system" includes, but is not limited to, the major pancreatic duct, the ductus hepatoparicreaticus, the papilla of Vater, the sphincter of Oddi, the accessory pancreatic duct, and the minor duodenal papilla.

The stenosis or/and obstruction may be located in the major pancreatic duct, in the ductus hepatopancreaticus, in the papilla of Vater, in the sphincter of Oddi, in the accessory pancreatic duct, or/and in the minor duodenal papilla.

In particular, the stenosis or/and obstruction may be located in the major pancreatic duct, in the accessory pancreatic duct, or/and in the minor duodenal papilla.

The obstruction may be caused by a concrement located in the pancreatic duct system, or/and by lithiasis, in particular pancreolithiasis. The concrement may be a pancreas calculus. The concrement may include carbonate.

The disease associated with stenosis or/and obstruction located in the pancreatic duct system may be lithiasis, pancreolithiasis, pancreatitis associated with concrement formation in the pancreatic duct system, wherein the pancreatitis may be chronic pancreatitis.

The disease or condition associated with stenosis or/and obstruction located in the pancreatic duct system may be cancer. In particular, the cancer causes a stenosis in the pancreatic duct system. The cancer may be cancer in the major pancreatic duct, in the ductus hepatopancreaticus, in the papilla of Vater, in the sphincter of Oddi, in the accessory pancreatic duct, or/and in the minor duodenal papilla. In particular, the cancer includes a tumor. The cancer may also be located in the circumventing pancreatic tissue.

In particular, the cancer may be located in the major pancreatic duct, in the accessory pancreatic duct, or/and in the minor duodenal papilla.

The cancer may be exocrine pancreatic cancer, or endocrine pancreatic cancer. The cancer may include a metastasis, for example a metastasis derived from a metastasizing cancer in another organ. In particular, the cancer may be selected from pancreatic cystadenoma, pancreatic cystadenocarcinoma, pancreatic adenoacanthoma, and secretory tumors, such as multiple endocrine neoplasia, insulinoma, glucagonoma, and somatostatinoma.

The stenosis or/and obstruction may cause pain.

The treatment may be a palliative treatment. Palliative treatment is indicated in patients suffering from cancer, in particular inoperable cancer, cancer in a terminal state, cancer which does not respond to anti-cancer treatment (for example by radiation or/and chemotherapy), the presence of metastases derived from a metastasizing cancer in another organ, wherein a metastasis causes a stenosis or/and obstruction in the pancreatic duct system, and wherein the cancer is in particular painful, in particular by formation of the stenosis or/and obstruction. More particular, the cancer forms a stenosis.

The treatment, in particular the palliative treatment may be continued for at least one month, at least two months, at least three months, at least four months, or at least six months.

Yet another aspect of the present invention is a pharmaceutical composition for use in a palliative treatment, said composition comprising lixisenatide or/and a pharmaceutically acceptable salt thereof and optionally a pharmaceutically acceptable carrier, diluent or/and auxiliary substance. The palliative treatment is the treatment of pain caused by a stenosis or/and obstruction in the pancreatic duct system. More particular, the palliative treatment includes the treatment of pain caused by cancer originating from stenosis or/and obstruction of the pancreatic duct system. The composition is a composition as described herein.

A further aspect of the present invention is a pharmaceutical composition for use in the treatment of pain, said composition comprising lixisenatide or/and a pharmaceutically acceptable salt thereof and optionally a pharmaceutically acceptable carrier, diluent or/and auxiliary substance. The pain is associated with stenosis or/and obstruction in the pancreatic duct system. The treatment of pain may be a palliative treatment, in particular a palliative treatment as described herein. The composition is a composition as described herein.

Also described herein is a method of treatment of a disease or condition associated with stenosis or/and obstruction in the pancreatic duct system, said method comprising administrating to a subject in need thereof a pharmaceutical composition comprising at least one GLP-1 agonist, and optionally a pharmaceutically acceptable carrier, diluent or/and auxiliary substance: In the method as described herein, the disease or condition may be any disease associated with stenosis or/and obstruction in the pancreatic duct system as described herein. The composition is a composition as described herein.

The patient or/and individual to be treated by the pharmaceutical composition or/and method as described herein may be a mammal, including humans and non-human mammals. A preferred patient or/and individual is a human.

In particular, the patient does not suffer from diabetes mellitus, such as type 1 or type 2 diabetes mellitus.

In particular, the patient is not obese. More particular, the patient's body mass index is below 30 kg/m$^2$ or below 27 kg/m$^2$.

In particular, the patient does not suffer from a CNS disorder, such as Alzheimer disease or Parkinson's disease.

As used herein, "antispasmodic activity" or "spasmolytic activity" of a GLP-1 agonist means that the tone of the smooth muscle, in particular in the bile system or/and the pancreatic duct system, is reduced by administration of a GLP-1 agonist. In particular, lixisenatide provides an antispasmodic or spasmolytic activity.

As used herein, the at least one GLP-1 agonist can be one, two, three, four, five or more GLP-1 agonists. In particular the at least one GLP-1 agonist can be one GLP-1 agonist. "GLP-1 agonist" is also termed "GLP-1 receptor agonist".

As used herein, the term "GLP-1 agonist" includes GLP-1, analogues and derivatives thereof, exendin-3, analogues and derivatives thereof, and exendin-4, analogues and derivatives thereof. Also included are substances which exhibit the biological activity of GLP-1.

The pharmaceutical composition as described herein can comprise one or more selected independently of one another from the group consisting of glucagon-like peptide-1 (GLP-1), analogues and derivatives of GLP-1, exendin-3, analogues and derivatives of exendin-3, exendin-4, analogues and derivatives of exendin-4.

GLP-1, exendin-3 or/and exendin-4, as used herein, include pharmacologically acceptable salts of GLP-1, exendin-3 or/and exendin-4.

It is preferred that the GLP-1 agonist is lixisenatide or/and a pharmaceutically acceptable salt thereof.

It is also preferred that the GLP-1 agonist is liraglutide or/and a pharmaceutically acceptable salt thereof.

GLP-1 analogues and derivatives are described in WO 98/08871, for example; exendin-3, analogues and derivatives of exendin-3, and exendin-4 and analogues and derivatives of exendin-4 can be found in WO 01/04156, WO 98/30231, US 5,424,286, in EP application 99 610 043.4, in WO 2004/005342 and WO 04/035623. The exendin-3 and exendin-4 described in these documents, and the analogues and derivatives thereof that are described there, can be used in the compositions as described herein as GLP-1 agonists. It is also possible to use any desired combination of the exendin-3 and exendin-4 described in these documents, and the analogues and derivatives described therein, as GLP-1 agonists.

The at least one GLP-1 agonist is preferably independently selected from the group consisting of exendin-4, analogues and derivatives of exendin-4, and pharmacologically acceptable salts thereof.

A further preferred GLP-1 agonist is an analogue of exendin-4 selected from a group consisting of:

H-desPro$^{36}$-exendin-4-Lys$_6$-NH$_2$ (desPro$^{36}$-exendin-4-Lys$_6$-NH$_2$, AVE0010, Lixisenatide),
H-des(Pro$^{36,37}$)-exendin-4-Lys$_4$-NH$_2$,
H-des(Pro$^{36,37}$)-exendin-4-Lys$_6$-NH$_2$,

and pharmacologically acceptable salts thereof.

A further preferred GLP-1 agonist is an analogue of exendin-4 selected from a group consisting of:

desPro$^{36}$ [Asp$^{28}$]exendin-4 (1-39),
desPro$^{36}$ [IsoAsp$^{28}$]exendin-4 (1-39),
desPro$^{36}$ [Met(O)$^{14}$, Asp$^{28}$]exendin-4 (1-39),
desPro$^{36}$ [Met(O)$^{14}$, IsoAsp$^{28}$]exendin-4 (1-39),
desPro$^{36}$ [Trp(O$_2$)$^{25}$, Asp$^{28}$]exendin-2 (1-39),
desPro$^{36}$ [Trp(O$_2$)$^{25}$, IsoAsp$^{28}$]exendin-2 (1-39),
desPro$^{36}$ [Met(O)$^{14}$Trp(O$_2$)$^{25}$, Asp$^{28}$]exendin-4 (1-39),
desPro$^{36}$ [Met(O)$^{14}$Trp(O$_2$)$^{25}$, IsoAsp$^{28}$]exendin-4 (1-39),

and pharmacologically acceptable salts thereof.

A further preferred GLP-1 agonist is an analogue of exendin-4 selected from a group as described in the paragraph above in which the peptide -(Lys)$_6$-NH$_2$ has been attached at the C-termini of the analogues of exendin-4.

A further preferred GLP-1 agonist is an analogue of exendin-4 selected from a group consisting of:

H-(Lys)$_6$-des Pro$^{36}$ [Asp$^{28}$]exendin-4(1-39)-Lys$_6$-NH$_2$
desAsp$^{28}$Pro$^{36}$, Pro$^{37}$, Pro$^{38}$ exendin-4(1-39)-NH$_2$,
H-(Lys)$_6$ des Pro$^{36}$, Pro$^{37}$, Pro$^{38}$ [Asp$^{28}$]exendin-4(1-39)-NH$_2$,
H-Asn-(Glu)$_5$ desPro$^{36}$, Pro$^{37}$, Pro$^{38}$ [Asp$^{28}$]exendin-4(1-39)-NH$_z$,
desPro$^{36}$, Pro$^{37}$, Pro$^{38}$ [Asp$^{28}$]exendin-4(1-39)-(Lys)$_6$-NH$_2$,
H-(Lys)$_6$-desPro$^{36}$, Pro$^{37}$, Pro$^{38}$ [Asp$^{28}$]exendin-4(1-39)-(Lys)$_6$-NH$_2$,
H-Asn-(Glu)$_5$-des Pro$^{36}$, Pro$^{37}$, Pro$^{38}$ [Asp$^{28}$]exendin-4(1-39)-(Lys)$_6$-NH$_2$,
H-(Lys)$_6$-desPro$^{36}$ [Trp(O$_2$)$^{25}$, Asp$^{28}$]exendin-4(1-39)-Lys$_6$-NH$_2$,
H-des Asp$^{28}$ Pro$^{36}$, Pro$^{37}$, Pro$^{38}$ [Trp(O$_2$)$^{25}$]exendin-4(1-39)-NH$_2$,
H-(Lys)$_6$-desPro$^{36}$, Pro$^{37}$, Pro$^{38}$ [Trp(O$_2$)$^{25}$, Asp$^{28}$]exendin-4(1-39)-NH$_2$,
H-Asn-(Glu)$_5$-desPro$^{36}$, Pro$^{37}$, Pro$^{38}$ [Trp(O$_2$)$^{25}$, Asp$^{28}$]exendin-4(1-39-NH$_2$,
des Pro$^{36}$, Pro$^{37}$, Pro$^{38}$ [Trp(O$_2$)$^{25}$, Asp$^{28}$]exendin-4(1-39)-(Lys)$_6$-NH$_2$,
H-(Lys)$_6$-des Pro$^{36}$, Pro$^{37}$, Pro$^{38}$ [Trp(O$_2$)$^{25}$, Asp$^{28}$]exendin-4(1-39)-(Lys)$_6$-NH$_2$,
H-Asn-(Glu)$_5$-des Pro$^{36}$, Pro$^{37}$ Pro$^{38}$ [Trp(O$_2$)$^{25}$, Asp$^{28}$]exendin-4(1-39)-(Lys)$_6$-NH$_2$,
H-(Lys)$_6$-des Pro$^{36}$ [Met(O)$^{14}$, Asp$^{28}$]exendin-4(1-39)-(Lys)$_6$-NH$_2$,
des Met(O)$^{14}$ Asp$^{28}$ Pro$^{36}$, Pro$^{37}$, Pro$^{38}$ exendin-4(1-39)-NH$_2$,
H-(Lys)$_6$-des Pro$^{36}$, Pro$^{37}$, Pro$^{38}$ [Met(O)$^{14}$, Asp$^{28}$]exendin-4(1-39)-NH$_2$,
H-Asn-(Glu)$_5$-des Pro$^{36}$, Pro$^{37}$, Pro$^{38}$ [Met(O)$^{14}$, Asp$^{28}$] exendin-4(1-39)-NH$_2$,
des Pro$^{36}$, Pro$^{37}$, Pro$^{38}$ [Met(O)$^{14}$, Asp$^{28}$]exendin-4(1-39)-(Lys)$_6$-NH$_2$,
H-(Lys)$_6$-desPro$^{36}$, Pro$^{37}$, Pro$^{38}$ [Met(O)$^{14}$, Asp$^{28}$]exendin-4 (1-39)-Lys$_6$-NH$_2$,
H-Asn-(Glu)$_5$ des Pro$^{36}$, Pro$^{37}$, Pro$^{38}$[Met(O)$^{14}$, Asp$^{28}$]exendin-4(1-39)-(Lys)$_6$-NH$_2$,
H-(Lys)$_6$-des Pro$^{36}$ [Met(O)$^{14}$, Trp(O$_2$)$^{25}$, Asp$^{28}$] exendin-4(1-39)-Lys$_6$-NH$_2$,
des Asp$^{28}$ Pro$^{36}$, Pro$^{37}$, Pro$^{38}$ [Met(O)$^{14}$, Trp(O$_2$)$^{25}$]exendin-4(1-39)-NH$_2$,
H-(Lys)$_6$-des Pro$^{36}$, Pro$^{37}$, Pro$^{38}$ [Met(O)$^{14}$, Trp(O$_2$)$^{25}$, Asp$^{28}$]exendin-4(1-39)-NH$_2$,
H-Asn-(Glu)$_5$-des Pro$^{36}$, Pro$^{37}$, Pro$^{38}$ [Met(O)$^{14}$, Asp$^{28}$] exendin-4(1-39)-NH$_2$,
des Pro$^{36}$, Pro$^{37}$, Pro$^{38}$ [Met(O)$^{14}$, Trp(O$_2$)$^{25}$, Asp$^{28}$]exendin-4(1-39)-(Lys)$_6$-NH$_2$,
H-(Lys)$_6$-des Pro$^{36}$, Pro$^{37}$, Pro$^{38}$ [Met(O)$^{14}$, Trp(O$_2$)$^{25}$, Asp$^{28}$]exendin-4(1-39)-(Lys)$_6$-NH$_2$,
H-Asn-(Glu)$_5$-des Pro$^{36}$, Pro$^{37}$, Pro$^{38}$ [Met(O)$^{14}$, Trp(O$_2$)$^{25}$, Asp$^{28}$] exendin-4(1-39)-(Lys)$_6$-NH$_2$,

and pharmacologically acceptable salts of these compounds.

A further preferred GLP-1 agonist is selected from a group consisting of Arg$^{34}$,Lys$^{26}$(N$^\varepsilon$($\gamma$-glutamyl(N$^\alpha$-hexadecanoyl)))GLP-1(7-37) [liraglutide] and pharmacologically tolerable salts thereof.

A further preferred GLP-1 agonist is Lixisenatide. Lixisenatide has the sequence of H-desPro$^{36}$-exendin-4-Lys$_6$-NH$_2$ (desPro$^{36}$-exendin-4-Lys$_6$-NH$_2$, AVE0010). This substance is published as SEQ ID No: 93 in WO 01/04156. Lixisenatide is a derivative of Exendin-4:

SEQ ID NO: 1: Lixisenatide (44 AS)
H-G-E-G-T-F-T-S-D-L-S-K-Q-M-E-E-E-A-V-R-L-F-I-E-W-L-K-N-G-G-P-S-S-G-A-P-P-S-K-K-K-K-K-K-NH$_2$

SEQ ID NO: 2: Exendin-4 (39 AS)
H-G-E-G-T-F-T-S-D-L-S-K-Q-M-E-E-E-A-V-R-L-F-I-E-W-L-K-N-G-G-P-S-S-G-A-P-P-P-S-NH$_2$

Preference is also given to pharmacologically tolerable salts of Lixisenatide.

The term "at least one GLP-1 agonist" includes combinations of the herein-described GLP-1·agonists which are used in the compositions of the invention, examples being any desired combinations of two or more GLP-1 agonists selected from the GLP-1 agonists described herein.

The at least one GLP-1 agonist is further preferably independently selected from exendin-4, H-desPro$^{36}$-exendin-4-Lys$_6$-NH$_2$, and Arg$^{34}$,Lys$^{26}$(N$^\varepsilon$($\gamma$-glutamyl(N$^\alpha$-hexadecanoyl)))GLP-1 (7-37) [liraglutide], and pharmacologically acceptable salts thereof.

The compositions as described herein may contain the GLP-1 agonist in an amount of 10 $\mu$g/ml to 20 mg/ml, preferably 25 $\mu$g/ml to 15 mg/ml. For the acidic to neutrally dissolved GLP-1 agonists the figures are preferably 20 $\mu$g/ml to 300 $\mu$g/ml, and for the neutral to basic agonists they are preferably 500 $\mu$g/ml to 10 mg/ml. For exendin-4 analogues, 20 $\mu$g/ml to 150 $\mu$g/ml are preferred.

The at least one GLP-1 agonist, in particular desPro$^{36}$Exendin-4(1-39)-Lys$_6$-NH$_2$ or/and the pharmaceutically acceptable salt thereof, may be administered to a subject in need thereof, in an amount sufficient to induce a therapeutic effect.

The at least one GLP-1 agonist, in particular desPro$^{36}$Exendin-4(1-39)-Lys$_6$-NH$_2$ or/and the pharmaceutically acceptable salt thereof, may be formulated with suitable pharmaceutically acceptable carriers, adjuvants, or/and auxiliary substances.

The at least one GLP-1 agonist, in particular desPro$^{36}$Exendin-4(1-39)-Lys$_6$-NH$_2$ or/and a pharmaceutically acceptable salt thereof, may be administered parenterally, e.g. by injection (such as by intramuscular or by subcutaneous injection). Suitable injection devices, for instance the so-called "pens" comprising a cartridge comprising the active ingredient, and an injection needle, are known. The at least one GLP-1 agonist, in particular desPro$^{36}$Exendin-4(1-39)-Lys$_6$-NH$_2$ or/and a pharmaceutically acceptable salt thereof, may be administered in a suitable amount, for instance in an amount in the range of 10 to 15 $\mu$g per dose or 15 to 20 $\mu$g per dose.

The at least one GLP-1 agonist, in particular desPro$^{36}$Exendin-4(1-39)-Lys$_6$-NH$_2$ or/and a pharmaceutically acceptable salt thereof, may be administered in a daily dose in the range of 10 to 20 $\mu$g, in the range of 10 to 15 $\mu$g, or in the range of 15 to 20 $\mu$g. The at least one GLP-1 agonist, in particular desPro$^{36}$Exendin-4(1-39)-Lys$_6$-NH$_2$ or/and a pharmaceutically acceptable salt thereof, may be administered by one injection per day.

The at least one GLP-1 agonist, in particular desPro$^{36}$Exendin-4(1-39)-Lys$_6$-NH$_2$ or/and a pharmaceutically acceptable salt thereof, may be provided in a liquid composition. The skilled person knows liquid compositions of Lixisenatide suitable for parenteral administration. A liquid composition of the present invention may have an acidic or a physiologic pH. An acidic pH preferably is in the range of pH 1 - 6.8, pH 3.5 - 6.8, or pH 3.5 - 5. A physiologic pH preferably is in the range of pH 2.5 - 8.5, pH 4.0 - 8.5, or pH 6.0 - 8.5. The pH may be adjusted by a pharmaceutically acceptable diluted acid (typically HCl) or pharmaceutically acceptable diluted base (typically NaOH).

The liquid composition comprising the at least one GLP-1 agonist, in particular desPro$^{36}$Exendin-4(1-39)-Lys$_6$-NH$_2$ or/and a pharmaceutically acceptable salt thereof, may comprise a suitable preservative. A suitable preservative may be selected from phenol, m-cresol, benzyl alcohol and p-hydroxybenzoic acid ester. A preferred preservative is m-cresol.

The liquid composition comprising the at least one GLP-1 agonist, in particular desPro$^{36}$Exendin-4(1-39)-Lys$_6$-NH$_2$ or/and a pharmaceutically acceptable salt thereof, may comprise a tonicity agent. A suitable tonicity agent may be selected from glycerol, lactose, sorbitol, mannitol, glucose, NaCl, calcium or magnesium containing compounds such as CaCl$_2$. The concentration of glycerol, lactose, sorbitol, mannitol and glucose may be in the range of 100 - 250 mM. The concentration of NaCl may be up to 150 mM. A preferred tonicity agent is glycerol.

The liquid composition comprising the at least one GLP-1 agonist, in particular desPro$^{36}$Exendin-4(1-39)-Lys$_6$-NH$_2$ or/and a pharmaceutically acceptable salt thereof, may comprise methionine from 0.5 $\mu$g/mL to 20 $\mu$g/mL, preferably from 1 $\mu$g /ml to 5 $\mu$g/ml. Preferably, the liquid composition comprises L-methionine.

The invention is further illustrated by the following figures and example.

**Figure 1:** Graphical illustration of the study design. For the physical examination, vital signs, laboratory testing, ECG, and AE assessments, refers to detailed Study Flow Chart. Abbr.: CCK-8 = cholecystokinin-8; IP = investigational product; EOS = end-of-study.

**Figure 2:** Example of gallbladder filling and emptying curve in a normal healthy subject under placebo and lixisenatide. Abscissa: time (minutes). Ordinate: counts per second (CPS).

**Figure 3:** Example of GBEF (%) in a normal healthy subject under placebo and lixisenatide. Abscissa: time (minutes). Ordinate: GBEF (%).

**Figure 4:** Mean and median individual plots - Pharmacodynamic population.

**Figure 5:** Mean (+SD) lixisenatide plasma concentrations (linear scale).

**Figure 6:** Mean lixisenatide plasma concentrations (semi-log scale).

**Figure 7:** Boxplots of GBEF at 30 min and 60 min (placebo on the right, lixisenatide 20 μg on the left). The time of assessment of GBEF corresponds to the time after start of CCK-8 infusion. Symbols: Box = Q1-Q3 Quartiles; + = Mean; Line inside Box = Median; Whisker length = Largest value $\leq$ 1.5(Q3-Q1); Star = outliers with Subject identifier. Abbreviations: GBEF = gallbladder ejection fraction; min = minutes.

## EXAMPLE

## SUMMARY

The example of the present invention refers to a randomized, double-blind, placebo-controlled, two-sequence, two-treatment cross-over study assessing the effect of a single subcutaneous injection of 20 μg lixisenatide on gallbladder motility in healthy male and female subjects. Gallbladder motility has been analysed by cholescintigraphy. Cholecystokinin (CCK-8) has been administrated 60 min after administration of a single dose of placebo or 20 μg lixisenatide and followed immediately by a single dose of $^{99m}$Tc mebrofenin (a $^{99m}$Tc-labeled iminodiacetic acid derivative). The procedure can be summarized as follows. By placing a radiation-sensitive camera over the subject's abdomen, a "picture" of the liver, bile ducts, and gallbladder can be obtained that corresponds to where the radioactive bile has migrated. After injection of lixisenatide or placebo and $^{99}$Tc injection, hepatic frame images were obtained at 1 frame/min for 60 min. After gallbladder visualization at 60 min, 0.02 μg/kg CCK-8 was administered via constant infusion pump for 60 min. Image acquisition was continued for at least an additional 60 min following CCK-8 infusion.

In the placebo group, CCK-8 induced a decline of $^{99m}$Tc counts recorded from the gallbladder, indicating a release of bile from the gallbladder via bile ducts into the duodenum. Surprisingly, by subcutaneous administration of a single dose of 20 μg lixisenatide in healthy subjects, this effect of CCK-8 is largely reduced (see exemplary filling and emptying curves in Figure 2). The amount of $^{99m}$Tc remains high in the gallbladder, indicating that only a small fraction of bile has been released. The effect has been quantified as follows. In the placebo group, CCK-8 induces an increase of the gallbladder ejection fraction (GBEF, or ejection fraction EF, i.e. the volume fraction of bile ejected from the gallbladder,) up to about 85%, as expected from the physiological action of CCK. In the lixisenatide group, the ejection fraction surprisingly remains below 40% (see exemplary GBEF curve in Figure 3, averages given in Figure 7 and Table 9) after CCK-8 administration. Under lixisenatide, 13/24 (54%) subjects had a GBEF at 60 min of below 40% compared to 1/24 (4%) under placebo. The subcutaneous administration of a single dose of 20 μg lixisenatide in healthy subjects significantly reduced gallbladder emptying expressed as gallbladder ejection fraction (GBEF) in response to CCK-8 compared to placebo at 60 min by 45.8%.

# SYNOPSIS

| Study center: | 1 |
|---|---|

| Publications (reference): None. |
|---|

| Phase of development: 1 |
|---|

**Objectives:**

<u>Primary Objective</u>: To assess the effect of 20 µg lixisenatide on gallbladder (GB) emptying expressed as GB ejection fraction (GBEF) at 60 minutes induced by a continuous infusion of 0.02 µg/kg.h cholecystokinin (CCK8).

<u>Secondary Objectives</u>: To assess GBEF at 30 minutes. To assess the pharmacokinetic profile of a single subcutaneous injection of 20 µg lixisenatide.

**Methodology:**

Single-center, double-blind, randomized, placebo-controlled, single-dose, 2period, 2treatment, 2sequence, crossover study

| **Number of subjects:** | Planned: 24 subjects (20 evaluable subjects) |
|---|---|
| | Randomized: 24 |
| | Treated: 24 |
| **Evaluated:** | pharmacodynamic: 24 |
| | Safety: 24 |
| | Pharmacokinetics: 24 |

**Diagnosis and criteria for inclusion:**

Male and female subjects aged 35 to 65 years with a body mass index between 18.0 and 35.0 kg/m$^2$; female subjects postmenopausal or surgically sterile for at least 3 months prior to the time of screening. Subjects with GB and liver abnormalities detected by ultrasonography were excluded.

**Investigational product:** Lixisenatide

Dose: 20 µg (in 200 µL sterile aqueous solution)

Administration: subcutaneous injection using a pen-type injector (OptiClik®)

Batch number: FRA01282/ 40C008/C1005517

**Duration of treatment:** 1 day each in treatment Period 1 and 2

**Duration of observation:** 7 to 42 days (ie, from informed consent to end-of-study evaluation)

**Reference therapy:** Placebo

Dose: 200 µL sterile aqueous solution

Administration: subcutaneous injection using a pen-type injector (OptiClik)

Batch number: FRA01419/ 40C006/C1005518

**Noninvestigational product:** technetium99m ($^{99m}$Tc) mebrofenin/Cholediam®

Dose: ≤60 MBq $^{99m}$Tc mebrofenin

Administration: intravenous injection

Batch number: FRA01419/ 40C006/C1005518

**Noninvestigational product:** Cholecystokinin8 (CCK8)

Dose: 0.02 µg/kg Kinevac® (sincalide)

Administration: infusion for 60 minutes

Batch number: C1008567

**Criteria for evaluation:**

pharmacodynamic:

Cholescintigraphy: GB emptying expressed as GBEF, which is the percentage change of net GB counts after CCK8 administration.

$$GBEF\ (\%) = \left[ \frac{(net\ GB\ counts\ before\ CCK\ \text{-}\ 8\ infusion) - (net\ GB\ counts\ at\ 30\ or\ 60\ min)}{net\ GB\ counts\ before\ CCK\ \text{-}\ 8\ infusion} \right] \times 100$$

Primary endpoint is the GBEF at 60 minutes induced by a continuous infusion of 0.02 µg/kg cholecystokinin8 (CCK8). Secondary endpoint is the GBEF at 30 minutes.

Safety: Adverse events, vital signs, electrocardiogram (ECG, automatic reading), standard clinical laboratory assessments (biochemistry and hematology)

Pharmacokinetics: Lixisenatide plasma concentration, pharmacokinetic parameters ($C_{max}$, $T_{max}$, $AUC_{last}$, $AUC_{t1\text{-}t2}$, AUC, $t_{1/2Z}$)

**Pharmacokinetic sampling times and bioanalytical methods:** To determine plasma concentrations of lixisenatide, blood was collected predose, and 0.5, 1, 1.5, 2, 3, 4, 6, 8, 10, and 12 hours after injection of lixisenatide.

**Statistical methods:**

**Pharmacodynamics:**

The primary endpoint, GBEF at 60 minutes, was analyzed using a linear mixed effects model. Differences between treatment groups and 95% confidence intervals (95% CI) were estimated within the framework of the linear mixed effects model. Noninferiority was demonstrated if the upper limit of the 2-sided 95% confidence interval for the difference between the 2 treatment groups (placebo minus lixisenatide) was less than 0.20 or 20% (absolute difference).

For the secondary endpoint, GBEF at 30 minutes, differences between treatment groups and 95% confidence intervals were estimated within the framework of the same linear mixed effects model as above.

Within-subject, between-subject, and total standard deviations for GBEF at 60 minutes and at 30 minutes were estimated, with 90% CIs, by equating observed and expected means squares within a linear mixed effects model framework.

Descriptive statistics and summary and individual plots are also provided.

**Pharmacokinetics:**

Lixisenatide pharmacokinetics were summarized using descriptive statistics.

**Safety and tolerability:**

The safety analysis was based on review of individual values (clinically significant abnormalities) and descriptive statistics by treatment. For adverse events, frequencies of treatment-emergent adverse events (TEAEs) classified by the Medical Dictionary for Regulatory Activities (MedDRA version 13.0) system organ class and preferred term were tabulated by treatment. All adverse events were listed.

**Pharmacokinetics:**

Lixisenatide pharmacokinetics were summarized using descriptive statistics.

**Safety and tolerability:**

The safety analysis was based on review of individual values (clinically significant abnormalities) and descriptive statistics by treatment. For adverse events, frequencies of TEAEs classified by MedDRA (version 13.0) system organ class and preferred term were tabulated by treatment. All adverse events were listed.

**Summary:**

pharmacodynamic results:

All 24 subjects who were randomized completed the two study periods and were included in the pharmacodynamic analysis.

Mean (SEM) GBEFs (%) at 30 and 60 minutes after placebo administration were 59.80% (5.67) and 84.95% (4.20), respectively, which is in agreement with values expected for healthy subjects. Thirty and 60 minutes after a single administration of lixisenatide mean GBEFs (%) were 17.97 (3.35) and 39.01 (5.85). Under lixisenatide 22 out of 24 subjects had a GBEF at 60 minutes that was lower than the GBEF under placebo at 60 minutes.

The estimate of the mean difference between placebo and lixisenatide for the primary endpoint (GBEF at 60 minutes) was 45.8% (95% CI: 29.92; 61.68). The upper limit of the confidence interval was greater than 20%, indicating that the non-inferiority of lixisenatide versus placebo was not demonstrated.

Statistical analysis GBEF at 60 minutes - Pharmacodynamic population

| Parameter | Comparison | Mean[a] of Placebo | Mean[a] of Lixisenatide 20 µg | Difference | | |
|---|---|---|---|---|---|---|
| | | | | Mean[b] | Lower 95% CI | Upper 95% CI |
| GBEF at 60 min (%) | Placebo vs. Lixisenatide 20 µg | 84.53 | 38.73 | 45.80 | 29.92 | 61.68 |

The time of assessment of GBEF corresponds to the time after start of CCK-8 infusion
LSM=least square means, CI=confidence interval
Number of subjects: N=24 under Placebo, N=24 under Lixisenatide 20 µg
[a]Mean is provided by LSM
[b]Mean difference = LSM (Placebo) - LSM (Lixisenatide 20 µg)

The estimate of the mean difference between placebo and lixisenatide was 41.43% (95% CI: 28.64; 54.23).

Statistical analysis GBEF at 30 minutes – Pharmacodynamic population

| Parameter | Comparison | Mean[a] of Placebo | Mean[a] of Lixisenatide 20 µg | Difference | | |
|---|---|---|---|---|---|---|
| | | | | Mean[b] | Lower 95% CI | Upper 95% CI |
| GBEF at 30 min (%) | Placebo vs. Lixisenatide 20 µg | 58.24 | 16.81 | 41.43 | 28.64 | 54.23 |

The time of assessment of GBEF corresponds to the time after start of CCK-8 infusion
LSM=least square means, CI=confidence interval
Number of subjects: N=24 under Placebo, N=24 under Lixisenatide 20 µg
[a]Mean is provided by LSM
[b]Mean difference = LSM (Placebo) - LSM (Lixisenatide 20 µg)

Safety results:

There were no serious adverse events or withdrawals from the study due to a TEAE. Under lixisenatide, 4/24 (16.7%) subjects experienced at least 1 TEAE compared to 1/24 (4.2%) subjects under placebo. All TEAEs were of mild or moderate intensity.

The TEAEs in subjects on lixisenatide were mainly gastrointestinal disorders, such as nausea (3/24) and vomiting (1/24). One subject experienced an episode of mild vomiting that occurred about 5 hours after the lixisenatide administration, and about 4 hours after the commencement of the CCK infusion. The symptom lasted about 5 minutes and resolved without treatment. There were no adverse events suggestive of an allergic reaction. One subject experienced an injection site hematoma 1 day after lixisenatide administration. The hematoma was of mild intensity and gradually resolved over the following 11 days.

No PCSAs were observed for liver function.

Prolonged QTc was observed for two male subjects. One subject who received lixisenatide during Period 2 had a QTc of 452 msec (rechecked: 455 msec) at the end-of-study visit (baseline: 418 msec). Another subject who received placebo during Period 1 had a QTc of 459 ms on Day 1 at Period 2 (rechecked value at D1: 436 ms).

Pharmacokinetic results: After subcutaneous dosing of 20 µg lixisenatide, the mean peak-exposure ($C_{max}$) was 104 pg/mL and appeared after 2 hours (median). The overall exposure (AUC) as mean amounted to 634 pg*h/mL, and the mean exposure during the interval for the primary endpoint for gallbladder emptying ($AUC_{12h}$) was 87.3 pg*h/mL.

**Conclusions**

In this placebo-controlled crossover study, subcutaneous administration of a single dose of lixisenatide 20 µg in healthy subjects significantly reduced GB emptying expressed as GBEF(%) in response to CCK-8 at 30 and 60 minutes. Non-inferiority of lixisenatide versus placebo after 60 minutes of CCK-8 infusion was not demonstrated.

Treatment with lixisenatide resulted in maximum plasma concentrations 2 hours after injection. The overall exposure (AUC) as mean was 634 pg*h/mL, and the mean exposure during the interval for the primary endpoint for gallbladder emptying ($AUC_{12h}$) was 87.3 pg*h/mL.

Lixisenatide was overall well tolerated and was assessed to be safe in the 24 subjects studied. The most frequent adverse event was nausea. None of the adverse events was severe or serious.

## 1 LIST OF ABBREVIATIONS AND DEFINITION OF TERMS

| | |
|---|---|
| Ab | antibody |
| AE | adverse event |
| AEPM | adverse events with pre-specified monitoring |
| ALT | alanine aminotransferase |
| ARAC | Allergic Reaction Assessment Committee |
| AUC | area under the concentration time curve |
| CCK | cholecystokinin |
| CCK-8 | terminal amino acid fragment of cholecystokinin |
| CI | confidence interval |
| $C_{max}$ | maximum observed plasma concentration |
| CRF | case report form |
| CT | computed tomography |
| CV | coefficient of variation |
| D | day |
| DBP | diastolic blood pressure |
| ECG | electrocardiogram |
| FDA | United States Food and Drug Administration |
| GB | gallbladder |
| GBEF | gallbladder ejection fraction |
| GLP | glucagon-like peptide |
| HCL | hydrochloric acid |
| HIV | human immunodeficiency virus |
| LLOQ | lower limit of quantification |
| MedDRA | Medical Dictionary for Regulatory Activities |
| MRI | magnetic resonance imaging |

| NaOH | sodium hydroxide |
|---|---|
| P | period |
| PCSA | potentially clinically significant abnormality |
| QC | quality control |
| QRS | QRS complex on electrocardiogram (ventricular depolarization) |
| QTc | QT interval automatically corrected for heart rate |
| QTcF | QT interval, Fridericia correction |
| SAE | serious adverse event |
| SBP | systolic blood pressure |
| SD | standard deviation |
| SEM | standard error of the mean |
| SOC | system organ class |
| $^{99m}Tc$ | technetium99m |
| TEAE | treatment-emergent adverse event |
| $t_{max}$ | time to reach Cmax |
| ULN | upper limit of normal |

## 2 ETHICAL CONSIDERATIONS

### 2.1 INDEPENDENT ETHICS COMMITTEE OR INSTITUTIONAL REVIEW BOARD

The protocol was submitted to independent ethics committees and/or institutional review boards for review and written approval.

### 2.2 ETHICAL CONDUCT OF THE STUDY

The protocol complied with recommendations of the 18th World Health Congress (Helsinki, 1964) and all applicable amendments. The protocol also complied with the laws and regulations, as well as any applicable guidelines, of the country where the study was conducted (Great Britain).

### 2.3 SUBJECT INFORMATION AND CONSENT

Informed consent was obtained prior to the conduct of any study-related procedures. The subject informed consent form was modified according to local regulations and requirements.

## 3 INTRODUCTION

Lixisenatide is a glucagon-like peptide 1 (GLP1) receptor agonist exendin analog being developed for treatment of type 2 diabetes.

Cases of acute pancreatitis have been reported in patients treated with GLP1 agonists, and the causality is still unclear. There is an ongoing discussion as to whether pancreatitis is a potential class effect of GLP1 treatment or whether the cases may have been overvalued. One hypothesis is that GLP1 agonists may change sphincter of Oddi motility due to gastric distension, predisposing patients to gallbladder (GB) sludge or gallstone formation and thus pancreatitis.

Cholecystokinin-stimulated cholescintigraphy was first described more than 3 decades ago and is used routinely to calculate gallbladder ejection fraction (GBEF) in studies on biliary dynamics and gallbladder motility. Cholescintigraphy is performed after administration of technetium-99m ($^{99m}Tc$) labeled iminodiacetic acid analogs. These compounds have a high affinity for hepatic uptake and are readily excreted into the biliary tract and concentrated in the gallbladder. A fatty meal or exogenous cholecystokinin is then used as a stimulus for induction of gallbladder emptying. A low GBEF has been considered as evidence of impaired gallbladder motor function that, in the absence of lithiasis, can identify patients with primary gallbladder dysfunctions and sphincter of Oddi obstruction. Since GBEF is variable depending on the dose and duration of cholecystokinin8 (CCK8, terminal amino acid fragment of CCK) infusion, many different values have been used to define abnormal gallbladder function; increasing evidence has suggested that longer infusions (30 to 60 minutes) are superior to shorter infusions (1 to 3 minutes) for accurate quantification of GBEF.

The aim of the present study was to assess the effect of a single subcutaneous injection of lixisenatide on stimulated gallbladder emptying (as an indirect measure of a potential impact on the sphincter of Oddi) expressed as GBEF induced by CCK8.

**4 STUDY OBJECTIVES**

**Primary objective**

- To assess the effect of 20 μg lixisenatide on gallbladder (GB) emptying expressed as GB ejection fraction (GBEF) at 60 minutes induced by a continuous infusion of 0.02 μg/kg h cholecystokinin (CCK8)

**Secondary objectives**

- To assess GBEF at 30 minutes

- To assess the pharmacokinetic profile of a single subcutaneous injection of 20 μg lixisenatide

**5 INVESTIGATIONAL PLAN**

**5.1 DESCRIPTION OF OVERALL STUDY DESIGN AND PLAN**

This was a single-center, double-blind, randomized, placebo controlled, single dose, 2period, 2-sequence, 2treatment crossover study comparing lixisenatide injection with saline injection (placebo) in healthy male and female subjects.

Subjects were admitted to the unit the morning on Day 1. On Day 1, after an overnight fast with ad libitum access to water, they were prepared for cholescintigraphy. Subjects received a subcutaneous dose of 20 μg of lixisenatide or placebo according to randomization, followed immediately by an intravenous bolus of the radiolabel. After $^{99m}$Tc injection, images were obtained for 60 minutes. After gallbladder visualization at 60 minutes, CCK8 was infused over 60 minutes; GB images were obtained for at least an additional 60 minutes following commencement of the CCK8 infusion.

Blood samples for determination of concentrations of lixisenatide were taken for up to 12 hours after injection of lixisenatide.

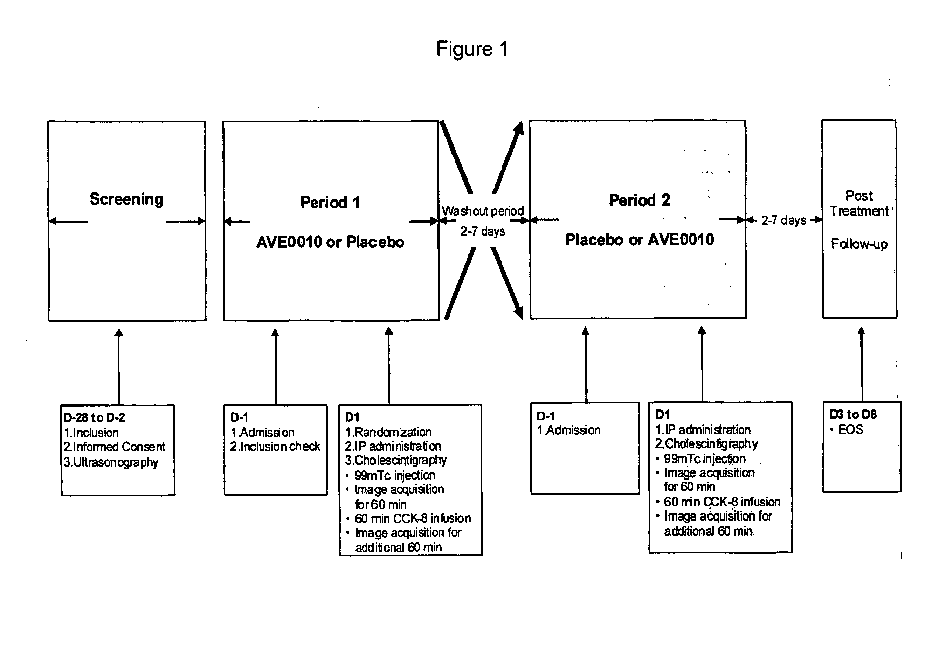

Subjects were randomized in a double-blinded manner to Sequence 1 or Sequence 2 (Figure 1):

Sequence 1

- Period 1: placebo

- Period 2: 20 μg lixisenatide

Sequence 2

- Period 1: 20 μg lixisenatide

- Period 2: placebo

The total duration of study participation for each subject was planned to be 7 to 42 days and consisted of:

- Screening: 2 to 28 days

- Treatment Period 1: 2 days (Day 1, Day 1) including 1 treatment day

- Washout: minimum 48 hours, maximum 7 days between doses (between Period 1, Day 1 and Period 2, Day 1)

- Treatment Period 2: 2 days (Day 1 to Day 1) including 1 treatment day

- End of study: 2 to 7 days after the last dosing

No interim analysis was planned. There were no amendments to the protocol.

The visit schedule is described in Table 1

## 5.2 DISCUSSION OF STUDY DESIGN AND CHOICE OF CONTROL GROUPS

This randomized, placebo-controlled, 2sequence, 2treatment crossover study was designed to assess the effect of a single dose of lixisenatide on gallbladder emptying. A crossover design was chosen to avoid the influence of between-subject variability.

The study included male and female subjects in order to match the type 2 diabetes target population. Although it has been observed that the majority of patients who present with Sphincter of Oddi dysfunction causing recurrent episodes of acute pancreatitis are female (1), in healthy subjects GBEF does not differ between men and women. Only postmenopausal females were included to avoid exposing females of childbearing potential to unnecessary radiation. Although significant impairment of GB emptying has been reported in obese type 2 diabetic patients (2), in healthy subjects no statistically significant correlation body mass index and GBEF has been observed. Therefore, male and female subjects with a body mass index up to 35 kg/m$^2$ were included in the study.

To maximize bile entry into the gallbladder by allowing maximum relaxation of the gallbladder and maximum contraction of the sphincter of Oddi (3), cholescintigraphy was conducted under fasted conditions. Radiolabel was injected directly after administration due to the lixisenatide $t_{max}$ of about 1.5 to 2 hours.

The rapid disappearance of lixisenatide from blood circulation when absorption is complete (mean peak plasma concentrations of approximately 100 pg/mL at about 1.5 hours after injection) enabled short washout periods of 2 days and an end-of-study visit within a week of the last dosing.

Since lixisenatide is a peptide that may potentially generate allergic reactions, the study employed an Allergic Reaction Assessment Committee assess allergic reactions or allergic-like reactions that occurred in the study.

## 5.3 SELECTION OF STUDY POPULATION

A total of 24 subjects were to be enrolled to have 20 evaluable subjects (with at least 30% of each gender randomized).

Subjects were included in the study according to the following criteria.

### 5.3.1 Inclusion criteria

**Demography**

I 1. Male or female subject, between 35 and 65 years inclusive. I 2. Body mass index between 18.0 and 35.0 kg/m$^2$ inclusive.

**Health Status**

I 3. Certified as healthy by a comprehensive clinical assessment (detailed medical history and complete physical examination).

I 4. Normal vital signs after 10 minutes resting in supine position:

- 95 mmHg < systolic blood pressure < 160 mmHg

- 45 mmHg < diastolic blood pressure < 90 mmHg

- 40 bpm < heart rate < 100 bpm

15. Normal standard 12 lead electrocardiogram (ECG) after 10 minutes resting in supine position; 120 ms < PR < 220 ms, QRS < 120 ms, QTc $\leq$ 430 ms if male, 450 ms if female

I 6. Laboratory parameters within the normal range, unless the Investigator considered an abnormality to be clinically irrelevant for healthy subjects; however, serum creatinine, alkaline phosphatase, hepatic enzymes (aspartate aminotransferase, alanine aminotransferase, bilirubin (unless the subject has documented Gilbert syndrome) were not to exceed the upper laboratory norm.

I 7. If female sterilized for more than 3 months or postmenopausal; menopause was defined as over the age of 60 years, or between 45 and 60 years being amenorrheic for at least 2 years with plasma follicle-stimulating hormone level >30 UI/L. Certified as healthy by a comprehensive clinical assessment (detailed medical history and complete physical examination).

**Regulations**

I 8. Must have given written informed consent prior to any procedure related to the study.

I 9. Covered by a Health Insurance System where applicable, and/or in compliance with the recommendations of the national laws in force relating to biomedical research.

I 10. Not under any administrative or legal supervision.

**5.3.2 Exclusion criteria**

**Medical history and clinical status**

E 1. Any history or presence of clinically relevant cardiovascular, pulmonary, gastrointestinal, hepatic, renal, metabolic, hematological, neurological, osteomuscular, articular, psychiatric, systemic, ocular, gynecologic (if female), or infectious disease, or signs of acute illness.

E 2. Frequent headaches and/or migraine, recurrent nausea and/or vomiting (more than twice a month).

E 3. Blood donation, any volume, within 3 months before inclusion.

E 4. Symptomatic postural hypotension, whatever the decrease in blood pressure, or asymptomatic postural hypotension defined by a decrease in systolic blood pressure $\geq$ 20 mmHg within 3 minutes when changing from the supine to the standing position.

E 5. Presence or history of drug hypersensitivity, or clinically significant allergic disease diagnosed and treated by a physician.

E 6. History or presence of drug or alcohol abuse (regular alcohol consumption >21 units per week in males and >14 units per week in females [1 Unit = ½ pint beer, a 25 mL shot of 40% spirit or a 125 mL glass of wine).

E 7. Smoking more than 5 cigarettes or equivalent per day, unable to stop smoking during the study.

E 8. Excessive consumption of beverages with xanthine bases (>4 cups or glasses per day).

**Interfering substance**

E 9. Any medication (including St John's Wort) within 14 days before the inclusion or within 5 times the elimination half-life or pharmacodynamic half-life of that drug, any vaccination within the last 28 days.

**General conditions**

E 10. Any subject who, in the judgment of the Investigator, was likely to be noncompliant during the study, or unable to cooperate because of a language problem or poor mental development.

E 11. Any subject in the exclusion period of a previous study according to applicable regulations.

E 12. Any subject who could not be contacted in case of emergency.

E 13. Any subject who was the Investigator or any subinvestigator, research assistant, pharmacist, study coordinator, or other staff thereof, directly involved in the conduct of the protocol.

**Biological status**

E 14. Positive reaction to any of the following tests: hepatitis B surface antigen, anti-hepatitis C virus antibodies, anti-human immunodeficiency virus 1 and 2 antibodies (antiHIV1 and antiHIV2 Ab).

E 15. Positive results on urine drug screen (amphetamines/methamphetamines, barbiturates, benzodiazepines, cannabinoids, cocaine, opiates).

E 16. Positive alcohol test.

**Specific to the study**

E 17. Acute diarrhea or constipation in the 7 days before the predicted first study day. If screening occurred >7 days before the first study day, this criterion was determined on first study day. Diarrhea was defined as the passage of liquid feces and/or a stool frequency of greater than 3 times per day. Constipation was defined as a failure to open the bowels more frequently than every other day.

E 18. Radiation exposure from clinical trials, including that from the present study, excluding background radiation but including diagnostic xrays and other medical exposures, exceeding 5 mSv in the last 12 months or 10 mSv in the last 5 years. No occupationally exposed worker, as defined in the Ionising Radiation Regulations 1999, was allowed to participate in the study.

E 19. Previous treatment with lixisenatide, exenatide (Byetta™) or other parenteral GLP1 agonists.

E 20. History of pancreatitis, chronic pancreatitis, gallbladder disease, pancreatectomy, stomach/gastric surgery, inflammatory bowel disease.

E 21. Presence of gall stones or clinically significant liver abnormalities on ultrasound scans.

**5.3.3 Removal of subjects from therapy or assessment**

Subjects could withdraw from the treatment if they decided to do so, at any time and irrespective of the reason, or subjects could be withdrawn based on the Investigator's decision.

Subjects experiencing a confirmed allergic reaction that was considered by the Investigator to be closely related to the administration of investigational product were to be withdrawn from further treatment.

Specific stopping rules were to be considered from screening to the end of the study. Administration of investigational product was to be stopped in case of QTcF (QTc, Fridericia correction) prolongation (automatic measurement: $\geq$ 500 ms), confirmed by a manual reading, and in the event of pregnancy. In addition, with any diagnosis of acute pancreatitis, treatment with investigational and other potentially suspect drugs was to be stopped and the subject was to be followed up clinically.

## 5.4 TREATMENTS

### 5.4.1 Investigational products

#### 5.4.1.1 Lixisenatide

- Pharmaceutical form: Sterile aqueous solution for subcutaneous injection in a 3-mL glass cartridge, containing the active ingredient 300 $\mu$g (ie, 100 $\mu$g/mL), glycerol, sodium acetate trihydrate, methionine, meta-cresol, HCL/NaOH, and water for injection.

- Route and method of administration: subcutaneous application using a pen-type injector (OptiClik®)

- Dose of drug per administration: 20 $\mu$g lixisenatide once in the morning on Day 1 in Period 1 or Period 2, according to the randomization schedule

- Investigational product was to be administered by deep subcutaneous injection in the left or right anterolateral abdominal wall

- For correct dosing, units of the OptiClik pen were to be set as follows: 20 $\mu$g lixisenatide = 20 Units on OptiClik (= 200 $\mu$L)

- Lixisenatide was provided by the Sponsor

#### 5.4.1.2 Placebo

- Pharmaceutical form: sterile aqueous solution for subcutaneous injection in a 3mL glass cartridge, containing sodium chloride, meta-cresol, HCL/NaOH, and water for injection

- Route and method of administration: subcutaneous application using a pen-type injector (OptiClik)

- Dose of drug per administration: 200 $\mu$L placebo once in the morning on Day 1 in Period 1 or Period 2, according to the randomization schedule

- Investigational product was to be administered by deep subcutaneous injection in the left or right anterolateral abdominal wall

- For correct dosing, units of the OptiClik pen were to be set as follows: 200 $\mu$L placebo = 20 Units on OptiClik.

- Placebo was provided by the Sponsor

The OptiClik pen-type injector was provided by the Sponsor to each subject for injection of investigational product. Needles (Ypsomed Optifine™ 8, 8 mm X 31G, Order no. 3100564) were purchased by the clinical unit. Dispensing materials were to be kept by the Investigator up to the full documented reconciliation performed with the Sponsor at the end of the study.

#### 5.4.1.3 Noninvestigational products

5.4.1.3.1 Radiopharmaceutical ($^{99m}$Tc mebrofenin/Cholediam)

The Cholediam® kit for preparation of $^{99m}$Tc mebrofenin injection was provided by Quotient Clinical and contained 40 mg mebrofenin and 0.6 mg stannous chloride dehydrate. The effective dose in this study was not to exceed 60 MBq (1.44 mSv) per period and 120 MBq (2.88 mSv) in total.

*5.4.1.3.2 Cholecystokinin8 (Kinevac/sincalide)*

Cholecystokinin8 (CCK8) was supplied as sincalide for injection (Kinevac®) by the Sponsor. Vials containing 5 μg sincalide were reconstituted with 5 mL sterile water. Then, 2.5 μg sincalide was diluted to 50 mL with saline in a 50 mL syringe and infused according to the following formula:

$$\text{Rate of infusion (mL/hr)} = \text{weight (kg)} \times 0.02 \text{ (CCK8 dose)} \times 20 \text{ (volume of solution containing 1 μg CCK8)}$$

**5.4.2 Identity of investigational products**

*5.4.2.1 Investigational products*

Investigational product was supplied by the Sponsor as a sterile aqueous solution for subcutaneous injection containing 300 μg/mL lixisenatide (100 μg/mL0, glycerol, sodium acetate trihydrate, methionine, meta-cresol, HCl/ NaOH, and water for injection in a 3-mL glass cartridge (batch number FRA01282/ 40C008/C1005517).
Placebo was supplied as a sterile aqueous solution for subcutaneous injection containing sodium chloride, meta-cresol, HCl/ NaOH, and water for injection (batch number FRA01419/ 40C006/C1005518).

*5.4.2.2 Noninvestigational products*

Technetium99m ($^{99m}$Tc) mebrofenin was supplied as a Cholediam for injection kit by Quotient Clinical; each vial contained 40 mg mebrofenin and 0.6 mg stannous chloride dehydrate in a sterile, pyrogen-free, freeze-dried solution under nitrogen (batch number FRA01419/40C006/C1005518).
Cholecystokinin-8 was supplied by the Sponsor as sincalide for injection (Kinevac) in vials containing 5 μg sincalide (batch number C 1008567).

**5.4.3 Method of assigning subjects to treatment groups**

The randomization treatment kit number list was generated centrally by sanofiaventis.
Before administration of investigational product on Day 1 of the first study period, subjects who complied with all inclusion/exclusion criteria were assigned:

• An incremental subject number according to the chronological order of inclusion.

• A treatment number (corresponding to one of the treatment sequences) in a preplanned order according to their subject numbers. Treatment numbers followed the randomization treatment kit number list. Subjects were to receive study medication according to their randomization treatment kit number.

Subjects were randomized to Sequence 1 or Sequence 2. The randomization ratio was 1:1 and was stratified by sex to ensure that at least 30% of each sex was treated in this study. For further details, refer to the study protocol.

**5.4.4 Selection of doses in the study**

The 20 μg dose of lixisenatide used in this study was considered to provide the best benefit-risk ratio and was well tolerated in both nondiabetic and diabetic populations, based on results of a Phase 2 dose-ranging study.
The risk associated with the maximum possible dose of radiation used was very small and was considered acceptable. The effective $^{99m}$Tc dose that each subject received did not exceed 60 MBq (1.44 mSv) for one administration and 120 MBq (2.88 mSv) for 2 administrations. This is in accordance with the Administration of Radioactive Substances Advisory Committee, which recommends that the $^{99m}$Tc dose not exceed 150 MBq for diagnostic procedures of the gallbladder and is only slightly higher than the average natural background radiation dose received in the United Kingdom each year (2.7 mSv).
Sincalide is the only CCK8 analogue approved by the US Food and Drug Administration (FDA). Although in published studies CCK8 doses varied from 0.01 to 0.5 μg/kg and the infusion duration from a bolus injection to durations of 1 to 60 minutes, short infusions caused abdominal cramps and nausea and made reproducible normal ranges for GB injection fraction difficult to achieve (4). In a study of 3 different CCK8 infusion methods in 60 subjects with a 0.02 μg/kg dose administered as a 15 minute, 30 minute, and 60 minute infusion, it was shown that a 60 minute infusion had the lowest variability in healthy subjects compared with shorter infusions of 15 and 30 minutes (5). Therefore, in this study, 0.02 $\mu$g/kg was infused for 60 minutes.

### 5.4.5 Selection and timing of dose for each subject

#### 5.4.5.1 Investigational products

##### 5.4.5.1.1 Lixisenatide

A 20 $\mu$g dose of lixisenatide was administered once in the morning in fasted conditions on Day 1 in Period 1 or Period 2, according to the randomization schedule. A 20 $\mu$g dose of lixisenatide corresponded to 20 units on the OptiClik pen (200 $\mu$L).

##### 5.4.5.1.2 Placebo

Placebo (200 $\mu$L) was administered once in the morning in fasted conditions on Day 1 in Period 1 or Period 2, according to the randomization schedule (20 units on the OptiClik pen).

#### 5.4.5.2 Noninvestigational products

##### 5.4.5.2.1 Radiopharmaceutical ($^{99m}$Tc mebrofenin/Cholediam)

A single dose of $^{99m}$Tc mebrofenin was infused intravenously in the morning on Day 1 of each period after an overnight fast of at least 10 hours, directly after administration of lixisenatide or placebo.

##### 5.4.5.2.2 Cholecystokinin8 (Kinevac/sincalide)

A single dose of CCK8 (0.02 $\mu$g/kg) was infused starting 60 minutes after administration of the radiolabel and for 60 minutes on Day 1 of each period.

### 5.4.6 Blinding procedures

All personnel involved in the study were blinded until database lock except for the bioanalyst and pharmacokineticist responsible for the sample analysis and pharmacokinetic evaluation.

Investigational product and placebo were indistinguishable and injection volumes were the same. Each treatment kit and the corresponding cartridge were labeled with a number generated by a computer program from sanofiaventis. The Investigator was not to have access to the randomization (treatment) code except when knowledge of the investigational product was essential for treating the subject.

ARAC members were to review and adjudicate allergic reactions or allergic-like reactions in a blinded manner.

### 5.4.7 Prior and concomitant therapy

Medications that were not to be used prior to inclusion are specified in Section 5.3.2.

Concomitant medication was not allowed during the study. However, if a specific treatment was required for any reason, an accurate record was to be kept on the appropriate record form, including the name of the medication (international nonproprietary name), daily dosage, and duration for such use.

### 5.4.8 Treatment compliance

Investigational product was administered under direct medical supervision. The actual dose and exact time of each administration was recorded in the case report form. A mouth inspection was to be performed to check for ingestion of investigational product by the subject.

### 5.5 PHARMACODYNAMICS, SAFETY AND PHARMACOKINETICS ASSESSMENTS

An overview of safety, pharmacokinetics, and pharmacodynamics assessments is presented in Table 1; a detailed schedule for each period is provided in Table 2.

**Table 1 - Study flow chart**

| Phase | Screening | Study Treatment Period 1 | | Washout Period[a] | Study Treatment Period 2 | | End-of-study |
|---|---|---|---|---|---|---|---|
| Day | D28 to D2 | D1 | D1 | 48 hours - 7 days | D1 | D1 | D3 to D8 |
| Informed consent | X | | | | | | |
| Institutionalization | | X | | | X | | |
| Discharge | | | X | | | X | |
| Visit at clinical site | X | | | | | | X |
| Ultrasonography | X | | | | | | |
| Inclusion exclusion criteria | X | X | | | | | |
| Medical / surgical history | X | | | | | | |
| Prior / concomitant medications | < | - | - | - | - | | > |
| Inclusion | | | X | | | | |
| Randomization | | | X | | | | |
| **Study treatment administration** | | | | | | | |
| Lixisenatide or placebo | | | X | | | X | |
| Radiolabel $^{99m}$Tc injection | | | X | | | X | |
| CCK8 infusion | | | X | | | X | |
| **Safety[b]** | | | | | | | |
| Physical examination | X | X | | | X | | X |
| Height | X | | | | | | |
| Body weight | X | | | | | | |
| Archival blood sample | | | X | | | | |
| Serology tests | X | | | | | | |
| Urine Drug Screen, alcohol breath test | X | X | | | X | | |
| Vital signs | X | X | | | X | | X |
| ECG | X | X | | | X | | X |
| Oral body temperature | X | X | | | X | | X |
| Blood Laboratory Examination | X | X | | | X | | X |
| Urinalysis | X | X | | | X | | X |
| β-HCG blood test (for females only) | X | X | | | | X | X |
| Plasma FSH[c] | X | | | | | | |
| Adverse event collection | < | | - | - | - | | > |
| **Pharmacokinetics** | | | | | | | |
| PK sample | | | X | | | X | |

(continued)

| Phase | Screening | Study Treatment Period 1 | | Washout Period[a] | Study Treatment Period 2 | | End-of-study |
|---|---|---|---|---|---|---|---|
| Day | D28 to D2 | D1 | D1 | 48 hours - 7 days | D1 | D1 | D3 to D8 |
| **Pharmacodynamics** | | | | | | | |
| Day | D28 to D2 | D1 | D1 | 48 hours - 7 days | D1 | 01 | D3 to D8 |
| Acclimatization session[d] | | X | | | | | |
| Cholescintigraphy (image acquisition) | | | X | | | X | |

[a] 27 days washout between doses
[b] Refer to Safety section for detailed safety investigations
[c] For females between 45 and 60 years being amenorrheic for at least 2 years
[d] Allow subjects to experience remaining stationary and supine under the camera.

D = day, PK = pharmacokinetic, CCK8 = cholecystokinin8, ECG = electrocardiogram, βhCG = beta human chorionic gonadotropin, FSH = follicle-stimulating hormone

**Table 2 - Period flow chart**

| Day | D1 | D1 | | | | | | | | | | | | D3 to D8 |
|---|---|---|---|---|---|---|---|---|---|---|---|---|---|---|
| Indicative clock time[a] | 08:00 | 7:00 | 8:00 | 8:30 | 9:00 | 9:30 | 10:00 | 11:00 | 12:00 | 14:00 | 16:00 | 18:00 | 20:00 | EoS visit |
| Indicative clock time[a] | 08:00 | 7:00 | 10:30 | 11:00 | 11:30 | 12:00 | 12:30 | 13:30 | 14:30 | 16:30 | 18:30 | 20:30 | 22:30 | |
| **PK/PD time (theoretical)[b]** | | | 0 | 0.5 | 1 | 1.5 | 2 | 3 | 4 | 6 | 8 | 10 | 12 | |
| Institutionalization | X | | | | | | | | | | | | | |
| Discharge | | | | | | | | | | | | | X | |
| Visit at clinical site | | | | | | | | | | | | | | |
| InclusionIExclusion criteria[c] | X | | | | | | | | | | | | | |
| Concomitant medications | < | - | - | - | - | - | - | - | - | - | - | - | - | > |
| Inclusion[c] | | X | | | | | | | | | | | | |
| Randomization[c] | | X | | | | | | | | | | | | |
| Meals | X[f] | | | | | | | X[f] | | | | | | |
| Study treatment administration | | | | | | | | | | | | | | |
| Lixisenatide or placebo | | | X | | | | | | | | | | | |
| Radiolabel $^{99m}$Tc injection | | | X[d] | | | | | | | | | | | |
| CCK8 infusion | | | | | X-------------------------------------X | | | | | | | | | |
| Safety[e] | | | | | | | | | | | | | | |
| Physical examination | X | | | | | | | | | | | | | X |
| Archival blood sample | | X[g] | | | | | | | | | | | | |
| Urine Drug Screen, alcohol breath test | X | | | | | | | | | | | | | |
| Vital signs | X | | | | | | | | | | | | | X |

| Day | D1 | D1 | | | | | | | | | | | | D3 to D8 |
|---|---|---|---|---|---|---|---|---|---|---|---|---|---|---|
| ECG | X | | | | | | | | | | | | | X |
| Oral body temperature | X | | | | | | | | | | | | | X |
| Blood Laboratory Examination | X | | | | | | | | | | | | | X |
| Urinalysis | X | | | | | | | | | | | | | X |
| β-HCG blood test (for females only) | X | | | | | | | | | | | | | X |
| Adverse event collection | < | - | - | - | - | - | - | - | - | - | - | - | - | > |
| **Pharmacokinetics** | | | | | | | | | | | | | | |
| Lixisenatide PK sample | | | P00[h] | P01 | P02 | P03 | P04 | P05 | P06 | P07 | P08 | P09 | P10 | |
| **Pharmacodynamics** | | | | | | | | | | | | | | |
| Acclimatization sessionc | X[i] | | | | | | | | | | | | | |
| Cholescintigraphy (image acquisition) | | | X--------------------------------------------------------------------X | | | | | | | | | | | |
| Indicative clock time[a] | 08:00 | 7:00 | 8:00 | 8:30 | 9:00 | 9:30 | 10:00 | 11:00 | 12:00 | 14:00 | 16:00 | 18:00 | 20:00 | EoS visit |
| Indicative clock time[a] | 08:00 | 7:00 | 10:30 | 11:00 | 11:30 | 12:00 | 12:30 | 13:30 | 14:30 | 16:30 | 18:30 | 20:30 | 22:30 | |

(continued)

| Day | D1 | D1 | | | | | | | | | | | | D3 to D8 |
|---|---|---|---|---|---|---|---|---|---|---|---|---|---|---|
| PK/PD time (theoretical) [b] | | | 0 | 0.5 | 1 | 1.5 | 2 | 3 | 4 | 6 | 8 | 10 | 12 | |

a Subjects dosing was staggered, with2 dosing times
b Time (hour/minute) is expressed in reference to the last administration of lixisenatide (T0H)
c Only Period 1
d Just after lixisenatide/placebo administration
e Refer to Safety section for detailed safety investigations
f Subjects were to have regular standard meals on Day 1. On Day 1 first meal after completion of scintigraphy for all subjects otherwise regular standard meals (snack, lunch and dinner)
g Only Period 1, after randomization
h Directly before dosing
i Subjects were allowed to remain stationary and supine under the camera
D = day, PK = pharmacokinetic, PD = pharmacodynamic, EOS = end-of-study, CCK8 = cholecystokinin8, ECG = electrocardiogram, βhCG = beta human chorionic gonadotropin

### 5.5.1 Pharmacodynamics assessments

Gallbladder emptying after stimulation by administration of CCK8 was expressed as GBEF, the percentage change of net GB counts after administration of the stimulus. GBEF was assessed at 30 and 60 minutes after the start of CCK8 administration according to the following formula:

$$\text{GBEF}_{\text{at timepoint t}}\ (\%) = \frac{\left(\text{net GB counts before CCK - 8 infusion}\right) - \left(\text{net GB counts at timepoint t}\right)}{\left(\text{net GB counts before CCK - 8 infusion}\right)} \times 100$$

#### 5.5.1.1 Pharmacodynamics measurements and timing

After an overnight fast, subjects received 20 μg lixisenatide subcutaneously and immediately afterward received an intravenous infusion of mebrofenin labeled with $^{99m}$Tc while lying supine underneath a large-field-of-view gamma camera. Hepatic phase images were obtained at 1 frame per minute for 60 minutes. After GB visualization at 60 minutes, 0.02 μg/kg CCK8 was administered via a constant infusion pump for 60 minutes. Image acquisition continued after the start of CCK8 infusion as 1minute frames for at least an additional 60 minutes until completion of the CCK8 infusion. Regions of interest were drawn around the gallbladder and background (adjacent normal liver) on a frame displaying a clear image of the gallbladder, and a background-corrected time-activity curve was generated. Due to the short duration of the assessment period, no correction for radioactive decay was performed.

#### 5.5.1.2 Primary pharmacodynamic variable(s)

The primary pharmacodynamic variable was GBEF at 60 minutes after start of the CCK8 infusion on Period 1, Day 1 and Period 2, Day 2.

#### 5.5.1.3 Secondary pharmacodynamic variables

The secondary pharmacodynamic variable was GBEF at 30 minutes after start of the CCK8 infusion on Period 1, Day 1 and Period 2, Day 2. GBEF was also collected every 2 minutes during the study.

### 5.5.2 Safety variables and timing of assessment

Safety was monitored via:

- adverse events spontaneously reported by the subjects or observed by the Investigator;

- clinical laboratory tests (biochemistry, hematology, urinalysis);

- electrocardiogram (ECG) recordings (heart rate; PR, QRS, QTintervals; QTc)

- vital signs measurements (heart rate, systolic and diastolic blood pressure; weight, height [for calculation of body mass index], body temperature);

- physical examinations

Clinically significant abnormalities (if any) were monitored until resolution or until clinically stable.
Serology (hepatitis B surface antigen, hepatitis C antibodies, antiHIV1 and antiHIV2 antibodies) was performed at screening only. A urine drug screen and alcohol breath test were performed at screening and on Day 1 of each period.

#### 5.5.2.1 Adverse events

All adverse events, regardless of seriousness or relationship to the investigational product, from signature of the informed consent form until the end-of-study visit, were to be recorded. For each adverse event, the Investigator was to specify the date of onset, intensity, action taken with regard to the investigational product, corrective treatment given, and outcome and was to provide an assessment as to whether there was a reasonable possibility that the adverse event was related to the investigational product.
A serious adverse event (SAE) is any untoward medical occurrence that at any dose:

- results in death; or

- is life-threatening; or

- requires inpatient hospitalization or prolongation of existing hospitalization; or

- results in persistent or significant disability/incapacity; or

- is a congenital anomaly/birth defect; or

- is a medically important event.

*5.5.2.1.1 Adverse events with pre-specified monitoring*

Adverse events requiring pre-specified monitoring (AEPMs) were defined as adverse events (serious or nonserious) that were to be monitored, documented, and managed in a pre-specified manner described in the protocol.
The Sponsor was to be notified immediately for:

- ALT increases $\geq$ 2 x upper limit of normal (ULN)

- QTc $\geq$ 500 ms

- Pregnancy

- Symptomatic overdose

- Pancreatitis and/or increase of pancreatic enzymes (amylase, lipase) >2 x ULN

AEPMs without immediate notification were:

- Asymptomatic overdose

- Local tolerability

- Skin reactions if score was $\geq$ 1 according to the scoring system described in Appendix C of the protocol. The protocol specified that a score of $\geq$ 3 was to be reported as an adverse event and a dermatologist was to be consulted in case of an irritation score of 3 or 4. This information was corrected in a Note to File (2 November 2009) to reporting of skin reactions for scores of $\geq$ 1 and a dermatologist consult for a score $\geq$ 4.

- Allergic or allergic-like reaction

**5.5.2.2 Laboratory safety parameters**

Standard clinical laboratory parameters (biochemistry, hematology, urinalysis) were measured at screening, on Day 1 of each period, and at the end-of-study visit (Table 1). Additional tests could be performed during the study according to the medical judgment of the Investigator. Blood samples were obtained in fasted conditions.

- Biochemistry

  - Plasma/serum electrolytes: sodium, potassium, calcium, chloride

  - liver function: ALT, aspartate aminotransferase, gamma-glutamyl transferase, alkaline phosphatases, total and conjugated bilirubin

  - metabolism: total cholesterol, triglycerides, glucose, albumin, total proteins

  - potential muscle toxicity: creatine phosphokinase

- renal function: urea, creatinine

- pancreas function: amylase, lipase

- Hematology

    - red blood cell count, hematocrit, hemoglobin, and platelets

    - white blood cell count with differential (neutrophils, lymphocytes, basophils, monocytes, and eosinophils)

    - international normalized ratio and activated partial thromboplastin time

- Urinalysis: dipstick proteins, glucose, erythrocytes, leucocytes (quantitative tests to be performed if positive), ketone bodies, pH

***Laboratory safety parameters with pre-specified monitoring***

The following laboratory abnormalities were to be monitored, documented, and managed.

- Neutropenia

- Thrombocytopenia

- Acute renal insufficiency

- Suspicion of rhabdomyolysis

*5.5.2.3 Other safety parameters*

**Vital signs**

Heart rate, blood pressure (systolic and diastolic measurements), and body temperature were measured at screening, Day 1 of each period, and at the end-of-study visit. Heart rate and blood pressure were obtained after 10 minutes in the supine resting position and also after 3 minutes in the standing position.

**Electrocardiogram**

A standard 12lead ECG was recorded after at least 10 minutes in the supine position (10second recording at 25 mm/s, 10 mm/mV). Electrocardiogram parameters derived from automatic measurements were HR, PR, QRS, QT, and QTc.

**Physical examination**

Physical examination included heart and respiratory auscultation; peripheral arterial pulse; pupil, knee, Achilles, and plantar reflexes; peripheral lymph node examination; and abdominal examination.

**5.5.3 Pharmacokinetics assessments and timing**

*5.5.3.1 Pharmacokinetic measurements and timing*

The sampling times for blood collection can be found in the Period Flow Chart (Table 2).

The bioanalytical method used for measuring lixisenatide in plasma samples is described briefly in Table 3.

**Table 3 - Bioanalytical method**

| Analyte | Lixisenatide |
| --- | --- |
| Matrix | Plasma |
| Analytical Technique | double-antibody sandwich ELISA |

(continued)

| Analyte | Lixisenatide |
|---|---|
| Lower Limit of Quantification | 12 pg/ml |
| Assay Range | 12 - 220 pg/mL |
| Assay Volume | 100 $\mu$L |

Abbreviations: ELISA = enzyme linked immunosorbent assay

All plasma samples from subjects who had been treated with lixisenatide were analyzed. From subjects who had received placebo, only the sample taken 1.5 hours postdose (P03) were to be analyzed by the bioanalyst, who was unblinded prematurely for this.

### 5.5.3.2 Pharmacokinetic variables

Table 4 lists the main pharmacokinetic parameters, which were determined based on plasma concentrations of lixisenatide. Partial AUCs during the analysis of gallbladder emptying were added in order to be able to explore the respective influence of the exposure.

**Table 4 - List of pharmacokinetic parameters and definitions**

| Parameters | Definition/Calculation |
|---|---|
| $C_{max}$ | Maximum observed plasma concentration |
| $t_{max}$ | Time to reach $C_{max}$ |
| $AUC_{last}$ | Area under the concentration time curve up to last measurable timepoint |
| $AUC_{t1t2}$ | Area under the concentration time curve in the interval t1 - t2 |
| AUC | Area under the concentration time curve extrapolated to infinity |
| $t_{1/2z}$ | Terminal elimination half-life associated with the terminal slope (Az) determined according to the following equation: $t_{1/2z} = 0.693/\lambda z$, where $\lambda z$ is the slope of the regression line of the terminal phase of the plasma concentration versus time curve in semi-logarithmic scale (h). |

### 5.5.4 Appropriateness of measurements

Standard measurements appropriate to assess the objectives were used in the study. The primary endpoint, GBEF, is commonly used to assess gall bladder emptying, and cholecystokinin-stimulated cholescintigraphy is used routinely for calculation of GBEF in the study of biliary dynamics and gallbladder motility.

### 5.6 DATA QUALITY ASSURANCE

Regular site monitoring ensured the quality of trial conduct. Management of clinical trial data was performed according to the following rules and procedures. Data entry, verification, and validation were carried out using standard computer software (Oracle® Clinical version 4.5.1); data were stored in an Oracle database on a digital VMS computer. A double-entry method was used to ensure that the data (except comments) were transferred accurately from the CRFs to the database. Moreover, every modification in the database could be traced using an audit trail. A data checking plan was established to define all automatic validation checks, as well as supplemental manual checks, to ensure data quality. All discrepancies were researched until resolved.

Sanofi-aventis conducted an Investigator meeting to develop a common understanding of the clinical study protocol, case report form, and study procedures as well as individual site initiation meeting.

### Pharmacokinetic data handling and data quality assurance

Demographic data, date and time of administration, date and time of sampling, and concentrations were transferred electronically to the pharmacokinetic database from the Oracle Clinical and Watson databases. The transfer of the concentration data into Watson was quality control (QC)-checked; no discrepancies were determined. Pharmacokinetic parameters given in all tables are computer-generated. Concentration values below the lower limit of quantification (LLOQ) were treated as zero in calculating mean values for the concentrations; for calculating pharmacokinetic parameters, they were treated as zero only if appearing before $C_{max}$, otherwise as 'missing'.

Mean calculations and their associated statistics were generated from unrounded numbers and may differ slightly from those values which would have been determined using rounded numbers. Once final pharmacokinetic analysis was performed, pharmacokinetic parameters were transferred electronically to the Biostatistics Department for further statistical. Concentration and pharmacokinetic parameter values were rounded to 3 significant figures in all tables of the report.

All raw data from the bioanalytical and pharmacokinetic sections of this study are held in the appropriate archive files.

Examples of gallbladder filling and emptying curves and GBEF (%) in a normal healthy subject under placebo and lixisenatide are provided (Figures 2 and 3).

**5.7 STATISTICAL METHODS PLANNED IN THE PROTOCOL AND DETERMINATION OF SAMPLE SIZE**

**5.7.1 Statistical and analytical plans**

Details of statistical methods are summarized as follows. The analysis of clinical data was performed under the responsibility of the sanofiaventis Biostatistics and Programming Department, using SAS® (SAS/Unix V9.2, SAS Institute, NC USA). The statistical analysis of pharmacokinetic parameters was performed by the Drug Disposition Department, using Pharmacokinetic Data Management System (PKDMS) (in-house software version 2.0 with WinNonlin Professional® version 5.2.1).

*5.7.1.1 Analysis of pharmacodynamic variables*

All pharmacodynamic analyses were performed using the pharmacodynamic population.

*5.7.1.1.1 Description of pharmacodynamic variables*

The pharmacodynamic endpoints are the GBEF measured by cholescintigraphy at 60 minutes (primary variable) and 30 minutes (secondary variable) induced by a continuous infusion of 0.02 $\mu$g/kg CCK8. GBEF, provided by Quotient Clinical, is equal to the percentage change of net GB counts after administration of the stimulus.

*5.7.1.1.2 Primary analysis*

GBEF at 60 minutes was analyzed using a linear mixed-effects model:

$$\mathrm{GBEF}_{\mathrm{at\ 60\ min}} = \mathrm{Sequence} + \mathrm{Period} + \mathrm{Sex} + \mathrm{Treatment} + \mathrm{Error}$$

with fixed terms for Sex, Sequence ("Lixisenatide - Placebo" versus "Placebo - Lixisenatide"), period (1 versus 2), and Treatment (Lixisenatide versus Placebo), and with an unstructured R matrix of treatment(i,j) variances and covariances for subject-within-sequence blocks, using SAS PROC MIXED.

Differences between treatment groups and corresponding 95% CIs were estimated within the linear mixed effects model framework. Noninferiority was demonstrated if the upper limit of the 2sided 95% CI for the absolute difference of GBEF between the 2 treatment groups (placebo minus lixisenatide) was less than 0.20 (or 20%).

It was explored graphically if GBEF at 60 minutes was normally distributed. In case of obvious deviation from the normal distribution, a nonparametric method was planned to be used.

*5.7.1.1.3 Secondary analysis/analysis of secondary variables*

**Analysis of the secondary endpoint**

For GBEF at 30 minutes, the same linear mixed effects model was carried out as described above to estimate the differences between treatment groups and corresponding 95% CIs. Likewise, a nonparametric approach was planned to be carried out if needed.

**Descriptive statistics and plots**

GBEF at 30 and 60 minutes were summarized by treatment group and listed by subject, sequence, and visit.

The same descriptive statistics are provided by sex.

Boxplots for both endpoints are provided by treatment group; individual plots were also produced.

Additionally, GBEF data every 2 minutes after the start of CCK8 infusion were plotted individually by subject, summarized by median and mean plots by treatment group, and listed.

**Variance components**

Within-subject, between-subject, and total standard deviations for GBEF at 30 and 60 minutes were estimated by equating observed and expected means squares within the following linear mixed effects model framework:

$$\mathrm{GBEF}_{\mathrm{at\ 30\ or\ 60\ min}} = \mathrm{Sequence} + \mathrm{Period} + \mathrm{Sex} + \mathrm{Treatment} + \mathrm{Subject(Sequence)} + \mathrm{Error}$$

with fixed terms for Sex, Sequence ("Lixisenatide - Placebo" versus "Placebo - Lixisenatide"), period (1 versus 2), and Treatment (Lixisenatide versus Placebo), and a random effect for subject within sequence, using SAS PROC MIXED. The 90% CIs were computed using the simple $\chi^2$ method for the within-subject SD and the GraybillWang procedure for the total SD (6).

**Listings**

A listing of GB cholescintigraphy data (GBEF values at 30 and 60 minutes, dates and times of cholescintigraphy) are provided by subject and sequence.

***5.7.1.2 Analysis of safety data***

The safety evaluation was based on review of the individual values (clinically significant abnormalities), descriptive statistics (summary tables, graphics) and if needed on statistical analysis (appropriate estimations, confidence intervals). The safety analysis was conducted according to the sanofiaventis document "Summarizing and Reporting Clinical Pharmacology Trial Data."

All safety analyses were performed using the safety population.

For all safety data, the observation period was divided into 3 segments:

- The pretreatment period was defined as the time between when the subject gave informed consent and the first administration of investigational product in Period 1 (excluded).

- The on-treatment period, for each treatment period, was defined as the time from investigational product administration up to 2 days after the administration of investigational product.

- The posttreatment period was defined as the time after the on-treatment period to either the administration of investigational product in the next period (for Period 1) or to the end of the follow-up (for Period 2).

All safety analyses were based on the on-treatment phase.

The definition of potentially clinical significant abnormalities (PCSA) list used in the statistical analysis of laboratory parameters, vital signs, and ECG data was version 2.0, dated 14 September 2009.

*5.7.1.2.1 Adverse events*

5.7.1.2.1.1 Definitions

Adverse events were coded according to the Medical Dictionary for Regulatory Activities (MedDRA, version 13.0). They were classified into predefined standard categories according to chronological criteria:

- Pretreatment adverse events: adverse events that developed or worsened during the pretreatment phase

- Treatment-emergent adverse events (TEAE): adverse events that occurred or worsened during an on-treatment phase

- Posttreatment adverse events: adverse events that occurred during the posttreatment phase

TEAEs were assigned to the investigational product received at the time of adverse event onset (lixisenatide or placebo). If a TEAE developed on one treatment and worsened under a later treatment, it was considered treatment-emergent for both treatments.

If the start date (or time) of an adverse event was incomplete or missing, then the adverse event was considered as a TEAE in each period unless a partial date (or time) or comment showed it to be a pre or posttreatment event.

All adverse events reported in the study were listed on an individual basis with flags to indicate adverse event status. This listing was sorted by subject, treatment, onset date, and time. However, the analyses of adverse events focused on the TEAEs.

5.7.1.2.1.2 Treatment-emergent adverse events

The numbers and percentages of subjects with any TEAE, any serious TEAE, any severe TEAE, any TEAE leading to permanent treatment discontinuation, or any TEAE leading to death (only if any occurred) were summarized by treatment group.

Subjects presenting TEAEs were listed sorted by treatment group, primary system organ class (SOC, sorted by MedDRA order) and preferred term (PT).

TEAEs were summarized by treatment group, tabulating:

- The number and percent of subjects with at least 1 TEAE within each and over all SOC(s);

- The number and percent of subjects experiencing each preferred term in each SOC;

- The number of occurrences of all preferred terms within each and over all SOC(s);

- The number of occurrences of each preferred term in each SOC.

5.7.1.2.1.3 Deaths, serious and other significant adverse events

Any death, SAE, or other significant adverse event was listed, sorted by subject, onset date, and time.

5.7.1.2.1.4 Adverse events leading to treatment discontinuation

Any adverse event leading to permanent treatment discontinuation was listed, sorted by subject, onset date, and time.

*5.7.1.2.2 Allergic reactions*

5.7.1.2.2.1 Listings of allergic reactions

Any cases of events potentially related to an allergic reaction were documented as adverse events with detailed complementary information.

A listing of individual data (separate from the listing of all adverse events, see Section 5.7.1.2.1.1) is provided, sorted by subject, onset date and time, irrespective of the definition of the on-treatment phase, including particularly a description of the adverse event, symptoms of the adverse event, possible etiologies, actions taken, vital signs measurements (at outset, during reaction, and at recovery) and a description of the allergic or allergic-like event.

The assessment of all these cases by the Allergic Reaction Assessment Committee (ARAC) was also listed, including particularly whether the event reported constituted an allergic reaction and if it did, its diagnosis and severity grade.

All cases are described in detail in the Clinical Study Report.

5.7.1.2.2.2 Subject and family allergic medical history

Subject and family allergic medical history, to be documented for subjects with any occurrence of potential allergic reaction, was coded according to the MedDRA version 13.0 and listed by subj ect.

*5.7.1.2.3 Suspected pancreatitis*

Any cases of suspected pancreatitis were documented as adverse events with detailed complementary information.

A listing of individual data (separate from the listing of all adverse events, see <u>Section</u> 5.7.1.2.1.1) is provided, sorted by subject, onset date, and time, irrespective of the definition of the on-treatment phase, including particularly a description of the adverse event, values of amylase and lipase, the gastroenterologist's evaluation, and potential causes of the pancreatitis.

All cases are described in detail in the clinical study report.

*5.7.1.2.4 Clinical laboratory evaluations*

5.7.1.2.4.1 Hematology and biochemistry data

Clinical laboratory safety (hematology, biochemistry, and urinalysis) was assessed on Day 1 of treatment Periods 1 and 2 and at the end-of-study visit. According to the study schedule, these safety parameters were not planned to be assessed during the on-treatment period.

Baseline values were the values collected on Day 1 in each treatment period. If any of the scheduled baseline tests were repeated for any subject, the last rechecked values were considered as baseline, provided the tests were done before the first investigational product administration and under the same conditions (eg, fasting for glucose).

Raw data for amylase and lipase were summarized in descriptive statistics by treatment group and timepoint.

The following listings are provided:

- A listing of individual data from subjects with postbaseline potentially clinically significant abnormality (PCSA)s, sorted by function and time of measurement;

- A listing of all individual data for hematology and biochemistry, including rechecked values and data from unscheduled laboratory tests, by biological function and time of measurement. In these listings, individual data were flagged when lower or higher than the lower or upper laboratory limits and/or when reaching the absolute limit of PCSA criteria, when defined;

- A listing of liver function data for subjects experiencing at least one of the following situations:

  - ALT >3 x ULN and total bilirubin >2 x ULN during the study, with at least one of them being postfirst dose, irrespective of the definition for the on-treatment phase;

  - Conjugated bilirubin >35% of total bilirubin and total bilirubin >1.5 x ULN on the same postfirst dose sample, irrespective of the definition for the on-treatment phase.

- If any, a listing related to increase in ALT $\geq$ 2 x ULN is provided, including especially the information on drug intake, medical and surgical history, alcohol habits, trigger factors, and event details with associated signs and symptoms;

- A listing of out-of-normal range definitions.

5.7.1.2.4.2 Urinalysis data

All qualitative and quantitative urinary test results (dipstick), including rechecked values, were listed.

*5.7.1.2.5 Vital signs*

5.7.1.2.5.1 Heart rate and blood pressure

Heart rate and systolic and diastolic blood pressure (SBP and DBP) were measured after 10 minutes in the supine resting position and after 3 minutes in standing position on Day 1 of treatment periods 1 and 2 and at the end-of-study visit. According to the study schedule, these safety parameters were not planned to be assessed during the on-treatment period.

The values used as baseline were the D1 assessment values of each treatment period. If any of the scheduled baseline tests were repeated for any subject, the last rechecked values were considered as baselines, provided the tests were done before the first investigational product administration. For heart rate and blood pressures, raw data (supine and standing positions) were summarized in descriptive statistics, for each type of measurement (position), parameter, and timepoint.

The following listings are provided:

- All individual data, including unplanned and rechecked values, listed by type of measurement (supine, standing, orthostatic). In the listings, values were flagged when reaching the limits of the PCSA criteria, when defined;

- A separate listing of individual data from subjects with postbaseline PCSAs.

5.7.1.2.5.2 Body temperature

All individual data were listed.

5.7.1.2.6 *Electrocardiogram*

Automatic reading ECG was performed on Day 1 of each treatment period and at the end-of-study visit. According to the study schedule, no on-treatment assessment was planned.

Heart rate and PR, QRS, QT, and corrected QTintervals (QTc) from automatic readings of the 12-lead ECG were analyzed as raw parameter values; baseline values were the Day 1 values of each period. If any of the scheduled baseline tests were repeated for any subject, the rechecked values were considered as baseline, provided the tests were performed before the first drug administration.

For all parameters, raw data were summarized in descriptive statistics, by treatment group and timepoint.

The following listings were provided:

- All individual data, including rechecked values, listed by type of measurement. In the listings, values were flagged when reaching the limits of the PCSA criteria, when defined;

- A separate listing of individual data of subjects with any postbaseline PCSAs;

- A separate listing of the cardiac profile for subjects with prolonged QTc (>450 ms for males, >470 ms for females) or changes from baseline in QTc >60 ms (if any), using all postdose timepoints, if any;

- A listing of subjects with at least one abnormality in qualitative assessment (ie, abnormal ECG) after the first dosing.

### 5.7.1.3 Analysis of pharmacokinetic data

All pharmacokinetic analyses were performed using the pharmacokinetic population.

At least the following pharmacokinetic parameters were determined on the day of dosing from plasma concentration data of lixisenatide using noncompartmental methods: $C_{max}$, $t_{max}$, $AUC_{last}$, AUC, and $t_{1/2z}$.

Pharmacokinetic parameters were summarized by descriptive statistics (number of observations (N), arithmetic and geometric means, standard deviation (SD), standard error of the mean (SEM), coefficient of variation (CV %), median, minimum and maximum, and number of observations.

To support a PK/PD analysis, early partial AUCs were calculated ($AUC_{t1-t2}$), with tlt2 of 02h, 1-2h, and 1h30.

### 5.7.1.4 Pharmacokinetics/pharmacodynamics analysis

Not applicable.

### 5.7.2 Other statistical/analytical considerations

Not applicable.

### 5.7.3 Determination of sample size

With a 2 x 2 crossover design, a total of 20 completed subjects (10 per sequence) was required to demonstrate for GBEF at 60 minutes that lixisenatide was not inferior to placebo by more than a 20% absolute difference (noninferiority margin) with a power of 90%, if the true within-subject SD is 0.10% and assuming the true difference between placebo and lixisenatide is at most 0.09%. To allow for dropouts, 24 subjects were to be enrolled in the study.

## 6 STUDY SUBJECTS

### 6.1 DISPOSITION OF SUBJECTS

A total of 24 subjects were included, randomized, and exposed to the study treatment (Table 5), and all subjects completed the 2 study periods.

**Table 5 - Subject disposition**

|  | All |
| --- | --- |
| Randomized and treated | 24 |
| Did not complete the study treatment period | 0 |
|  |  |
| Subject's request for treatment discontinuation | 0 |

### 6.2 PROTOCOL DEVIATIONS

#### 6.2.1 Deviations relating to selected criteria and resulting in exclusion from the pharmacodynamic analyses

There were no protocol deviations that led to exclusion from the pharmacodynamic analyses. One deviation related to not meeting inclusion criterion I05 (QRS=122 msec) was not considered as clinically relevant.

#### 6.2.2 Randomization and dosing irregularities

There were no randomization irregularities during the study. All 24 subjects received investigational product (lixisenatide, placebo) and the radiolabel and CCK-8 were administered as planned.

Subject No. 826001012 did not seem to be exposed to lixisenatide in any trial periods according to his pharmacokinetic profiles. Several investigations were performed to rule out technical issues. These investigations were described in a Note to File.

- It was ruled out that another subject received lixisenatide instead of Subject No. 826001012; in this dosing group no other subject had an unusually high exposure to lixisenatide.

- The site confirmed in writing that administration was correctly performed. All cartridges were checked prior to use and drug accountability was complete.

- Cartridges 0000644 from Batch IP0000988 were re-analyzed and product identity of each cartridge was confirmed.

Since no plausible explanation could be found for this observation, the subject was included in the pharmacodynamic and pharmacokinetic analyses.

#### 6.2.3 Other deviations

No other important deviations were observed.

### 6.3 BREAKING OF THE BLIND

The blind was not broken during the study.

### 6.4 DATA SETS ANALYZED

The safety, pharmacodynamic, and pharmacokinetic populations comprised 24 subjects (Table 6).

**Table 6 - Analysis populations**

|  | All |
| --- | --- |
| Safety population | 24 |
| Pharmacokinetic population | 24 |
| Pharmacodynamic population | 24 |

## 6.5 DEMOGRAPHIC AND OTHER BASELINE CHARACTERISTICS

### 6.5.1 Demography

Demographic data for the safety population are summarized in Table 7. There were 15 male and 9 female subjects, aged between 35 and 62 years (mean age $\pm$SD: 47.8 $\pm$7.9 years), and a mean body mass index of about 26 kg/m$^2$. All subjects were of Caucasian origin except for 1 subject who was Black.

**Table 7 - Demographics and subject characteristics at baseline - Safety population**

|  | All (N=24) |
| --- | --- |
| Age (years) |  |
| Number | 24 |
| Mean (SD) | 47.8 (7.9) |
| Min : Max | 35 : 62 |
| Sex [n (%)] |  |
| Number | 24 |
| Male | 15 (62.5%) |
| Female | 9 (37.5%) |
| Race [n (%)] |  |
| Number | 24 |
| Caucasian/White | 23 (95.8%) |
| Black | 1 (4.2%) |
| Weight (kg) |  |
| Number | 24 |
| Mean (SD) | 77.75 (12.77) |
| Min: Max | 56.3 : 103.0 |
| BMI (kg/m$^2$) |  |
| Number | 24 |
| Mean (SD) | 26.21 (2.77) |
| Min : Max | 21.0 : 30.5 |

Demographic characteristics for male and female subjects are summarized in Table 8.

**Table 8 - Demographics and subject characteristics at baseline by gender - Safety population**

|  | Male (N=15) | Female (N=9) |
| --- | --- | --- |
| Age (years) |  |  |
| Number | 15 | 9 |
| Mean (SD) | 44.7 (6.7) | 52.8 (7.5) |
| Min: Max | 35 : 59 | 41:62 |

(continued)

| | Male (N=15) | Female (N=9) |
|---|---|---|
| Race [n (%)] | | |
| Number | 15 | 9 |
| Caucasian/White | 14 (93.3%) | 9 (100%) |
| Black | 1 (6.7%) | 0 |
| | | |
| Weight (kg) | | |
| Number | 15 | 9 |
| Mean (SD) | 85.27 (9.06) | 65.22 (6.54) |
| Min : Max | 67.2 : 103.0 | 56.3 : 73.7 |
| | | |
| BMI (kg/m$^2$) | | |
| Number | 15 | 9 |
| Mean (SD) | 26.92 (2.78) | 25.04 (2.48) |
| Min : Max | 21.0 : 30.5 | 21.2 : 27.7 |

**6.5.2 Medical history**

Medical history was obtained at screening for inclusion purposes only; no relevant medical history was recorded.

**6.5.3 Disease characteristics at baseline**

Not applicable. The study enrolled healthy subjects.

**6.5.4 Other baseline characteristics**

None

**6.5.5 Prior and/or concomitant medication**

There were no prior medications stopped before the study start. No concomitant medication was administered during the study.

**6.6 MEASUREMENT OF TREATMENT COMPLIANCE**

There were 24 subjects who received the radiolabel, CCK8 infusion, and treatment with either lixisenatide or placebo for 1 day during both trial periods, as planned. The duration of CCK-8 infusion corresponded to the protocol-specified duration of 60 minutes for all subjects.

**7 PHARMACODYNAMICS EVALUATION**

**7.1 PHARMACODYNAMICS RESULTS**

**7.1.1 Primary pharmacodynamic variable - gallbladder ejection fraction at 60 minutes**

The parametric estimate of the mean difference between placebo and lixisenatide for the primary endpoint (GBEF at 60 minutes) is 45.80% (95% CI: 29.92; 61.68). The upper limit of the confidence interval is greater than 20%, indicating that noninferiority of lixisenatide versus placebo is not demonstrated (Table 9).

One subject (No. 826001012) did not seem to be exposed to lixisenatide in any trial periods according to his pharmacokinetic profiles. No plausible explanation was possible and hence the data of this subject have been included in the pharmacodynamic population. However, for information purposes, exclusion of this subject from the analysis had minimal impact on the outcome.

**Table 9 - Statistical analysis of main endpoint: GBEF at 60 minutes - Pharmacodynamic population**

| Parameter | Comparison | Mean[a] of Placebo | Mean[a] of Lixisenatide 20 μg | Difference | | |
|---|---|---|---|---|---|---|
| | | | | Mean[b] | Lower 95% CI | Upper 95% CI |
| GBEF at 60 min (%) | Placebo vs. Lixisenatide 20 μg | 84.53 | 38.73 | 45.80 | 29.92 | 61.68 |

The time of assessment of GBEP corresponds to the time after start of CCK8 infusion
LSM=least square means, CI=confidence interval
Number of subjects: N=24 under Placebo, N=24 under Lixisenatide 20 μg
[a]Mean is provided by LSM
[b]Mean difference = LSM (Placebo) - LSM (Lixisenatide 20 μg)

### 7.1.1.1 Secondary analyses

### 7.1.2 Secondary pharmacodynamic variable

### 7.1.2.1 Gallbladder ejection fraction at 30 minutes

The parametric estimate of the mean difference between placebo and lixisenatide for the secondary endpoint (GBEF at 30 minutes) is 41.43% (95% CI: 28.64; 54.23) (Table 10).

Table 17 shows the GBEF at 30 minutes without data from the subject who seemed to be not exposed to lixisenatide in any trial period (see Section 7.1.1).

**Table 10 - Statistical analysis of secondary endpoint: GBEF at 30 minutes - Pharmacodynamic population**

| Parameter | Comparison | Mean[a] of Placebo | Mean[a] of Lixisenatide 20 μg | Difference | | |
|---|---|---|---|---|---|---|
| | | | | Mean[b] | Lower 95% CI | Upper 95% CI |
| GBEF at 30 min (%) | Placebo vs. Lixisenatide 20 μg | 58.24 | 16.81 | 41.43 | 28.64 | 54.23 |

The time of assessment of GBEF corresponds to the time after start of CCK8 infusion
LSM=least square means, CI=confidence interval
Number of subjects: N=24 under Placebo, N=24 under Lixisenatide 20 μg
[a]Mean is provided by LSM
[b]Mean difference = LSM (Placebo) - LSM (Lixisenatide 20 μg)

### 7.1.2.2 Descriptive statistics at 30 minutes and 60 minutes

Descriptive statistics for GBEF are summarized in Table 11.

The mean (SEM) GBEFs (%) at 30 and 60 minutes after placebo administration were 59.80 (5.67) and 84.95 (4.20), respectively. Thirty and 60 minutes after a single administration of lixisenatide, the mean GBEFs were 17.97 (3.35) and 39.01 (5.85), respectively (Table 11).

Box plots of GBEF at 30 and 60 minutes after placebo and lixisenatide administration are presented in Figure 7

**Table 11 - Descriptive statistics of GBEF at 60 min and 30 min - Pharmacodynamic population**

| | Raw data | | | | | | | | |
|---|---|---|---|---|---|---|---|---|---|
| | N | Mean | SD | SEM | Median | Min | Q1 | Q3 | Max |
| GBEF (%) at 60 min | | | | | | | | | |
| Placebo | 24 | 84.95 | 20.59 | 4.204 | 95.45 | 17.1 | 79.50 | 98.90 | 100.0 |
| Lixisenatide 20 μg | 24 | 39.01 | 28.66 | 5.849 | 33.85 | 0.0 | 18.00 | 50.35 | 96.7 |

(continued)

| Raw data | | | | | | | | |
| | N | Mean | SD | SEM | Median | Min | Q1 | Q3 | Max |
|---|---|---|---|---|---|---|---|---|---|
| GBEF (%) at 30 min | | | | | | | | | |
| Placebo | 24 | 59.80 | 27.76 | 5.666 | 57.75 | 10.0 | 42.60 | 84.20 | 97.7 |
| Lixisenatide 20 μg | 24 | 17.97 | 16.42 | 3.351 | 12.85 | 0.0 | 5.45 | 33.80 | 59.8 |

GBEF = Gallbladder ejection fraction The time of assessment of GBEF corresponds to the time after start of CCK-8 infusion

### 7.1.2.3 Descriptive statistics at 2 minutes

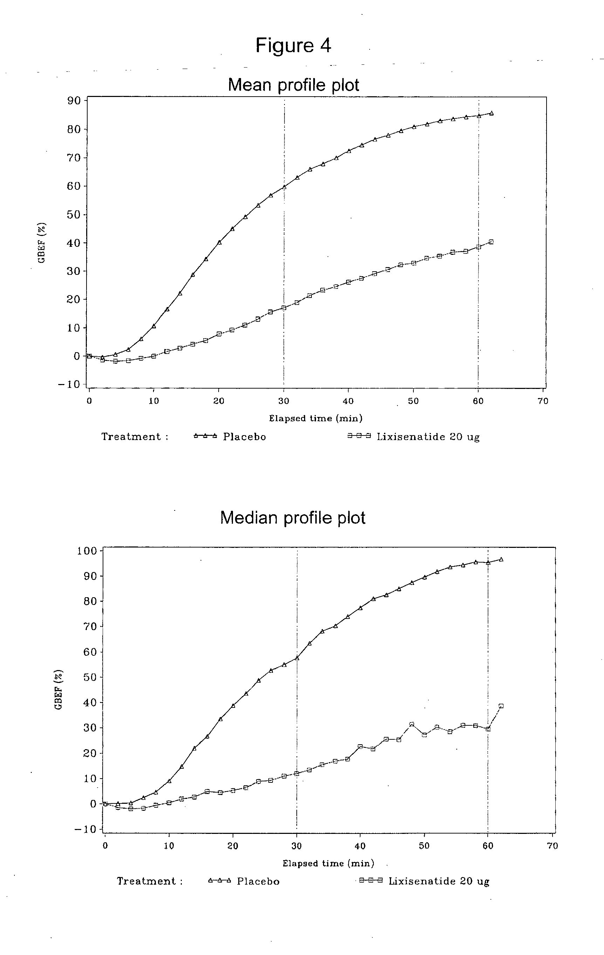

Plots of mean and median GBEF every 2 minutes during CCK-8 infusion under each treatment are provided in Figure 4. GBEF (%) for lixisenatide was lower than for placebo at all timepoints and increased more slowly than for placebo. Both curves showed a smooth increase over time.

### 7.1.3 Pharmacodynamic conclusions

A single administration of 20 μg lixisenatide significantly reduced GB emptying expressed as GBEF (%) in response to CCK8 compared to placebo at 60 minutes by 45.8% (absolute difference: 95% CI: 29.92; 61.68). Noninferiority of lixisenatide versus placebo was not demonstrated.

The design of this study and the sample size calculation were based on the methodology reported in published literature (5). Using the same dose and duration of infusion of CCK8, this published study reported that in normal healthy subjects the mean.GBEF percentages at 30 and 60 minutes were 64% ($\pm$23%) and 84% ($\pm$16%), respectively. In the PDY11431 study, the GBEF values under placebo were in line with what was expected and were at normal levels (~40% at 60 minutes) in 23 of the 24 subjects. The exception was Subject No. 826001001 who exhibited a GBEF of only 17% at 60 minutes after the start of CCK8 infusion following the administration of placebo. This does not appear to be indicative of consistent functional disorder in this subject, since under lixisenatide the GBEF at 60 minutes was 75.1 %.

Under lixisenatide, 22 out of 24 subjects had a GBEF at 60 minutes that was lower than the GBEF under placebo at 60 minutes; for another subject, GBEFs under lixisenatide and placebo were equal. GBEF at 60 minutes was below the lower limit of normal (~40%) in 13 out of 24 subjects.

## 8 SAFETY EVALUATION

### 8.1 EXTENT OF EXPOSURE

All 24 included subjects received either a single dose of 20 μg lixisenatide or placebo during each period of the study and completed the study as planned.

### 8.2 ADVERSE EVENTS

#### 8.2.1 Brief summary of adverse events

No severe TEAEs, treatment-emergent SAEs, or TEAEs leading to study treatment discontinuation were reported during the study.

On lixisenatide treatment, 4 out of 24 (16.7%) subjects experienced at least 1 TEAE compared to 1 out of 24 (4.2%) subjects treated with placebo. All TEAEs were of mild or moderate intensity.

There were no reported allergic adverse events that required a review by the Allergic Reaction Assessment Committee and no cases of suspected pancreatitis.

**Table 12 - Overview of adverse event profile: treatment-emergent adverse events - Safety population**

| | Placebo (N=24) | Lixisenatide 20 μg (N=24) |
|---|---|---|
| n(%) | n (%) | n(%) |
| Subjects with any TEAE | 1 (4.2%) | 4 (16.7%) |

(continued)

| n(%) | Placebo (N=24) n (%) | Lixisenatide 20 μg (N=24) n(%) |
|---|---|---|
| Subjects with any severe TEAE | 0 | 0 |
| Subjects with any treatment emergent SAE | 0 | 0 |
| Subjects with any TEAE leading to permanent treatment discontinuation | 0 | 0 |

TEAE: Treatment emergent adverse event, SAE: Serious adverse event N = Number of subjects treated within each group, n (%) = number and % of subjects with at least one TEAE in each category Note: An adverse event is considered as treatment emergent if it occurred from the time of the first investigational product (IP) administration of a period up to 2 days (included) after the last IP administration of the period.

### 8.2.2 Display of adverse events

The number and percentage of subjects with TEAEs are summarized in Table 13 by treatment group, primary system organ class, and preferred term. All adverse events, described both by the preferred term and the original term used by the Investigator, are provided..

**Table 13 - Number (%) of subjects with treatment-emergent adverse events by primary system organ class and preferred term - Safety population**

| Primary System Organ Class Preferred Term [n (%)] | Placebo (N=24) n (%) | Lixisenatide 20 μg (N=24) n (%) |
|---|---|---|
| Any class | 1(4.2%) | 4(16.7%) |
| Gastrointestinal disorders | 0 | 3 (12.5%) |
| Nausea | 0 | 3 (12.5%) |
| Vomiting | 0 | 1 (4.2%) |
| General disorders and administration site conditions | 0 | 1 (4.2%) |
| Injection site hematoma | 0 | 1 (4.2%) |
| Injury, poisoning and procedural complications | 1 (4.2%) | 0 |
| Corneal abrasion | 1 (4.2%) | 0 |

TEAE: Treatment-emergent adverse event, SOC:system organ class, PT: Preferred term MedDRA 13.0 N = Number of subjects treated within each group, n (%) = number and % of subjects with at least one TEAE in each category Note: Table sorted by SOC internationally agreed order and decreasing frequency of PT in Lixisenatide 20 μg group

Note: An adverse event is considered as treatment emergent if it occurred from the time of the first investigational product (IP) administration of a period up to 2 days (included) after the last IP administration of the period.

### 8.2.3 Analysis of adverse events

TEAEs in subjects receiving lixisenatide were mainly from the gastrointestinal disorders SOC. The most commonly reported TEAE was nausea, which was reported in 3 subjects (Table 13). All TEAEs were either mild or moderate in intensity.

One subject (No. 826001005) experienced an episode of mild vomiting that occurred about 5 hours after lixisenatide administration and 4 hours after commencement of CCK infusion and was assessed as possibly related to lixisenatide and CCK8 treatment. The symptoms lasted about 5 minutes and resolved without treatment.

One subject (No. 826001021) experienced an infection site hematoma 1 day after lixisenatide administration that was considered as possibly related to lixisenatide by the Investigator. The hematoma was of mild intensity and gradually resolved over the following 11 days.

One subject (No. 826001013) experienced abdominal pain 6 days after lixisenatide administration and was considered as a posttreatment adverse event. The subject was noted to have mild tenderness on deep inspiration and palpation at the right costal margin at the follow-up physical examination. The subject did not observe any abdominal discomfort apart from deep palpation. The remainder of the abdominal examination was normal as were safety laboratory evaluations. The symptoms were fully resolved 2 days later without treatment. This event was considered as possibly related to lixisenatide and CCK-8 administration by the Investigator.

No adverse event led to a global score of $\geq 3$ according to the evaluation of skin responses scale.

A total of 10 adverse events in 8 subjects were documented during the study: 1 pretreatment adverse event, 6 treatment-emergent adverse events, and 3 posttreatment adverse events. The most common adverse events were gastrointestinal disorders (5 adverse events), followed by general disorders (2 adverse events), injury, poisoning and procedural disorders (2 adverse events), and skin and subcutaneous tissues disorders (1 adverse event).

A listing of adverse events (preferred terms) per subject (treatment group) is given below.

- Subject No. 826001002: contusion (pretreatment)

- Subject No. 826001005: nausea, vomiting (lixisenatide)

- Subject No. 826001007: nausea (lixisenatide)

- Subject No. 826001009: catheter site swelling (placebo)

- Subject No. 826001013: abdominal pain (lixisenatide)

- Subject No. 826001015: corneal abrasion (placebo)

- Subject No. 826001021: injection site hematoma (lixisenatide)

- Subject No. 826001024: blood blister (placebo), nausea (lixisenatide)

## 8.3 DEATHS, SERIOUS ADVERSE EVENTS, AND OTHER SIGNIFICANT ADVERSE EVENTS

### 8.3.1 Deaths

No deaths were reported during the study.

### 8.3.2 Serious adverse events

There were no serious adverse events reported during the study.

### 8.3.3 Adverse events leading to withdrawal and other significant adverse events

There were no adverse events that led to withdrawal from the study, and no significant adverse events were reported.

## 8.4 CLINICAL LABORATORY EVALUATIONS

A subject listing of all laboratory values and all possibly clinically significant abnormalities (PCSA) laboratory values is provided.

### 8.4.1 Laboratory value over time

Only individual data for laboratory values were analyzed.

**8.4.2 Individual** subject **changes in laboratory values**

There were a few PCSAs in potassium, glucose, neutrophils, and eosinophils on Period 2, Day -1 and at the end-of-study visit. A few subjects had lipase values >2 x ULN but rechecked values were within the normal range. There were no reports of PCSAs for liver function, renal function, platelets, or coagulation.

**8.4.3 Individual clinically relevant abnormalities in laboratory values**

No clinically relevant abnormalities in laboratory values were reported during the study.

**8.5 VITAL SIGNS, PHYSICAL FINDINGS, AND OTHER SAFETY OBSERVATIONS**

**8.5.1 Vital signs**

Subject listings of all vital signs data are provided.

*8.5.1.1 Vital signs values over time*

Descriptive statistics for vital signs data are provided.

*8.5.1.2 Individual subject changes in vital signs*

One subject (No. 826001004) who was randomized in the sequence placebo-lixisenatide experienced an orthostatic decrease in SBP (standing-supine SBP $\leq$ 20 mm Hg) at the end-of-study visit. No other PCSAs were observed during the study.

*8.5.1.3 Individual clinically relevant abnormalities in vital signs*

No clinically relevant abnormalities in vital signs were reported during the study.

**8.5.2 Electrocardiograms**

Subject listings of all ECG data are provided.

*8.5.2.1 Electrocardiogram values over time*

Descriptive statistics for ECG data are provided.

*8.5.2.2 Individual subject changes in electrocardiogram*

There was no subject with a PCSA for QTc $\geq$ 500 ms (Table 14).
Subject No. 826001006, a 44year old male, had a normal QTc of 418 ms at baseline of Period 2. Four (4) days after he had received a single subcutaneous administration of 20 $\mu$g lixisenatide, he had an asymptomatic PCSA for prolonged QTc of 452 ms (rechecked value 455 ms) at the end-of-study visit, corresponding to an increase of 34 ms from baseline (Table 14).
Subject No. 826001017, a 43year old male in the treatment sequence placebo-lixisenatide, had an asymptomatic PCSA for prolonged QTc of 459 ms (rechecked value 436 ms) at baseline of Period 2. The QTc value at the end-of-study visit was normal (409 ms).

**Table 14 - Listing of subjects with prolonged QTc and/or delta QTc >60 ms (automatic reading) - Safety population**

| Visit | Theor. time | HR (bpm) Value | PR (ms) Value | QRS (ms) Value | QT (ms) Value | QTc (ms) Value |
|---|---|---|---|---|---|---|
| Subject=826001006 (Male / 44 years / 166 cm / 83.7 kg / 30.4 kg/m$^2$ / Caucasian/White) - Sequence=Placebo /Lixisenatide 20 $\mu$g | | | | | | |
| P2D1 | T24H | 64 B | 142 B | 96 B | 406 B | 418 B |

(continued)

| Visit | Theor. time | | HR (bpm) Value | PR (ms) Value | QRS (ms) Value | QT (ms) Value | QTc (ms) Value |
|---|---|---|---|---|---|---|---|
| Subject=826001006 (Male / 44 years / 166 cm / 83.7 kg / 30.4 kg/m$^2$ / Caucasian/White) - Sequence=Placebo /Lixisenatide 20 $\mu$g | | | | | | | |
| EOS | | | 89 | 136 | 82 | 372 | 452 ++ |
| EOS | | r | 81 | 134 | 82 | 392 | 455 ++ |
| Subject=826001017 (Male / 43 years / 183 cm / 88.0 kg / 26.3 kg/m$^2$ / Caucasian/White) - Sequence=Placebo /Lixisenatide 20 $\mu$g | | | | | | | |
| P2D1 | T24H | | 66 | 194 | 116 | 438 | 459 ++ |
| P2D1 | T24H | r | 56 B | 192 B | 110 B | 452 B | 436 B + |
| EOS | | | 46 | 198 | 110 | 468 | 409 |

PCSA: Potentially Clinically Significant Abnormalities (Version of 14-Sept2009) B = Baseline, r = rechecked values - or +/++ : Abnormal value reaching the lower or the 1st/2nd upper PCSA limit Note : A PCSA is considered to be on-treatment if it occurred from the time of the first investigational product (IP) administration of a period up to 2 days (included) after the last IP administration of the period.

### *8.5.2.3 Individual clinically relevant abnormalities in electrocardiogram*

No clinically relevant abnormalities in ECG values were reported during the study.

### 8.6 SAFETY CONCLUSIONS

Overall, the administration of 20 $\mu$g lixisenatide was well tolerated in healthy subjects. There were no serious adverse events and no withdrawals from the study due to TEAEs.

On lixisenatide treatment, 4 out of 24 (16.7%) subjects experienced at least 1 TEAE compared to 1 out of 24 (4.2%) subjects treated with placebo. All TEAEs were of mild intensity and resolved without corrective treatment. The TEAEs in subjects administered lixisenatide were mainly from the gastrointestinal disorders SOC, such as nausea (3 subjects out of 24) and vomiting (1 subject out of 24).

There were no clinically significant changes in laboratory parameters. No PCSAs were observed in liver and renal function.

There were no clinically significant findings in vital signs and ECG parameters. No subject had a QTc $\geq$ 500 ms. One male subject who received lixisenatide during Period 2 had a prolonged QTc of 452 ms corresponding to an increase from baseline of 34 ms at the end-of-study visit (4 days after lixisenatide administration). Another subject who received placebo during Period 1 had a QTc of +459 ms on Day -1 at Period 2. The QTc value at the endofstudy visit was normal (409 ms).

No adverse events related to an allergic reaction or suspected pancreatitis were reported.

### 9 PHARMACOKINETIC EVALUATION

### 9.1 PLASMA CONCENTRATIONS

The bioanalytical report for the lixisenatide assay will be available at a later date. Different from the original plan, erroneously all samples from the placebo arm were analyzed.

Individual lixisenatide plasma concentrations and descriptive statistics are provided. Individual lixisenatide plasma concentrations versus time curves are calculated, and superimposed curves for lixisenatide plasma concentrations versus time are calculated.

All blood samples were collected within $\pm$15% of the scheduled sampling times.

Except in one case, the samples taken prior to the lixisenatide dose had concentrations for lixisenatide below LLOQ (12 pg/mL)(for Subject No. 826001010 a value > LLOQ (19.9 pg/mL) was determined predose). In 2 cases subjects who received placebo showed isolated values >LLOQ (Subject No. 826001005 @ T1h30, 12.1 pg/mL and Subject No. 826001023 @ T10h, 18.2 pg/m) whereas the other measurable samples of their profiles were <LLOQ.

For Subject No. 826001012 in both trial periods (placebo and lixisenatide) all samples were below LLOQ, a reason for this could not be found. As a consequence, no pharmacokinetic parameters could be calculated for this subject.

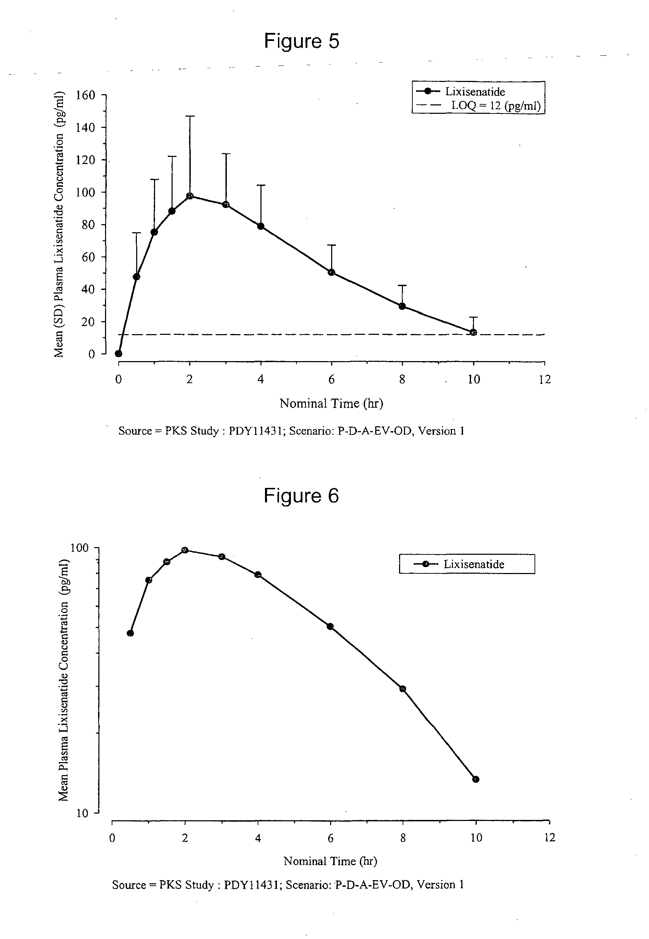

Mean (SD) plasma lixisenatide concentration-time profiles for the lixisenatide treatments are shown in Figure 5 (linear scale) and Figure 6 (semilog scale).

## 9.2 PHARMACOKINETIC PARAMETERS

Individual pharmacokinetic analyses of lixisenatide plasma pharmacokinetic data obtained after a single administration of 20 $\mu$g lixisenatide and the corresponding descriptive statistics are provided.

A summary of the descriptive statistics for lixisenatide pharmacokinetic data is given in Table 15.

### Table 15 - Pharmacokinetic data for lixisenatide

| Mean $\pm$ (Geometric Mean) [CV%] | SD |
|---|---|
| | Lixisenatide |
| N | 23[a] |
| $C_{max}$ (pg/ml) | 104 $\pm$ 48.7 (97.2) [46.7] |
| $t_{max\ b}$ (hr) | 2.00 (0.98 - 4.00) |
| $t_{1/2z}$ (h) | 2.62 $\pm$ 0.660 (2.55) [25.2] |
| $AUC_{last}$ (pg•h/ml) | 567 $\pm$ 196 (536) [34.5] |
| AUC (pg•h/ml) | 634 $\pm$ 189 (609) [29.8] |
| $AUC_{02h}$ (pg•h/ml) | 130 $\pm$ 54.9 (122) [42.2] |
| $AUC_{12h}$ (pg•h/ml) | 87.3 $\pm$ 36.5 (82.1) [41.9] |
| $AUC_{11.5h}$ (pg•h/ml) | 40.9 $\pm$ 16.4 (38.5) [40.2] |
| a No parameters could be calculated for 1 subject (Subject No. 826001012; see Section 9.1 | |
| b (Min - Max) | Median |

After subcutaneous dosing of 20 $\mu$g lixisenatide, the mean peak exposure ($C_{max}$) was 104 pg/mL and appeared after 2 hours (median). The overall exposure (AUC) as mean was 634 pg*h/mL, and the mean exposure during the interval for the primary endpoint for gallbladder emptying ($AUC_{12h}$) was 87.3 pg*h/mL.

## 9.3 PHARMACOKINETIC/PHARMACODYNAMIC RELATIONSHIP

Not applicable

## 9.4 PHARMACOKINETIC CONCLUSIONS

After subcutaneous dosing of 20 $\mu$g lixisenatide, the mean peak-exposure ($C_{max}$) was 104 pg/mL and appeared after 2 hours (median). The overall exposure (AUC) as mean was 634 pg*h/mL, and the mean exposure during the interval for the primary endpoint for gallbladder emptying ($AUC_{12h}$) was 87.3 pg*h/mL.

## 10 DISCUSSION AND OVERALL CONCLUSIONS

In this placebo-controlled crossover study, subcutaneous administration of a single dose of lixisenatide 20 $\mu$g in healthy subjects significantly reduced GB emptying expressed as GBEF(%) in response to CCK-8 at 30 and 60 minutes. Non-inferiority of lixisenatide versus placebo after 60 minutes of CCK-8 infusion was not demonstrated.

Treatment with lixisenatide resulted in maximum plasma concentrations 2 hours after injection. The overall exposure (AUC) as mean was 634 pg*h/mL, and the mean exposure during the interval for the primary endpoint for

gallbladder emptying ($AUC_{12h}$) was 87.3 pg*h/mL.

Lixisenatide was overall well tolerated and was assessed to be safe in the 24 subjects studied. The most frequent adverse event was nausea. None of the adverse events was severe or serious.

## 11 REFERENCES

1. Kakhi VR, Zakavi SY, Davoudi Y. Normal values of gallbladder ejection fraction using 99mTc-sestamibi scintigraphy after a fatty meal formula. J Gastrointestin Liver Dis. 2007 Jun;16(2):157-61. Pubmed PMID: 17592562.

2. Kao CH, Tsou CT, Wang SJ, Yeh SH. The evaluation of gallbladder function by quantitative radionuclide cholescintigraphy in patients with noninsulin-dependent diabetes mellitus. Nucl Med Common. 1993 Oct;14(10):868-72. PubMed PMID: 8233230.

3. Prandini N. Methods of measuring gallbladder motor functions - the need for standardization: scintigraphy. Dig Liver Dis. 2003 Jul;35 Suppl 3:S62-6. PubMed PMID: 12974513.

4. Krishnamurthy GT, Krishnamurthy S, Brown PH. Constancy and variability of gallbladder ejection fraction: impact on diagnosis and therapy. J Nucl Med. 2004 Nov;45(11):1872-7. PubMed PMID: 15534057.

5. Ziessman HA, Tulchinsky M, Lavely WC, Gaughan JP, Allen TW, Maru A, et al. Sincalide-stimulated cholescintigraphy: a multicenter investigation to determine optimal infusion methodology and gallbladder ejection fraction normal values. J Nucl Med. 2010 Feb;51(2):277-81. Epub 2010 Jan 15. PubMed PMID: 20080900.

6. Burdick R, Graybill F. (1992). Confidence intervals on variance components. Marcel Dekker, NY. ISBN 0-8247-8644-0..

## 12 SUPPORTIVE INFORMATION

**Table 16 - Statistical analysis of main endpoint: GBEF at 60 minutes - Pharmacodynamic population without Subject No. 826001012**

| Parameter | Comparison | Meana of Placebo | Meana of Lixisenatide 20 μg | Difference | | |
|---|---|---|---|---|---|---|
| | | | | Meanb | Lower 95% CI | Upper 95% CI |
| GBEF at 60 min (%) | Placebo vs. Lixisenatide 20 μg | 84.23 | 37.08 | 47.16 | 30.86 | 63.45 |

The time of assessment of GBEF corresponds to the time after start of CCK8 infusion
LSM=least square means, CI=confidence interval
Number of subjects: N=23 under Placebo, N=23 under Lixisenatide 20 μg
aMean is provided by LSM
bMean difference = LSM (Placebo) - LSM (Lixisenatide 20 μg)

**Table 17 - Statistical analysis of secondary endpoint: GBEF at 30 minutes - Pharmacodynamic population without Subject No. 826001012**

| Parameter | Comparison | Meana of Placebo | Meana of Lixisenatide 20 μg | Difference | | |
|---|---|---|---|---|---|---|
| | | | | Meanb | Lower 95% CI | Upper 95% CI |

(continued)

| Parameter | Comparison | Mean[a] of Placebo | Mean[a] of Lixisenatide 20 μg | Difference | | |
|---|---|---|---|---|---|---|
| | | | | Mean[b] | Lower 95% CI | Upper 95% CI |
| GBEF at 30 min (%) | P lacebo vs. Lixisenatide 20 μg | 58.78 | 16.32 | 42.45 | 29.29 | 55.62 |

The time of assessment of GBEF corresponds to the time after start of CCK8 infusion LSM=least square means, CI=confidence interval Number of subjects: N=23 under Placebo, N=23 under Lixisenatide 20 μg

[a]Mean is provided by LSM

[b]Mean difference = LSM (Placebo) - LSM (Lixisenatide 20 μg)

## Claims

1. A pharmaceutical composition for use in the treatment of a disease or condition, wherein said disease or condition is associated with stenosis or/and obstruction located in the pancreatic duct system, or/and in the circumventing pancreatic tissue, said composition comprising lixisenatide or/and a pharmaceutically acceptable salt thereof and optionally a pharmaceutically acceptable carrier, diluent or/and auxiliary substance.

2. The pharmaceutical composition for use of claim 1, wherein the stenosis or/and obstruction is located in the major pancreatic duct, in the ductus hepatopancreaticus, in the papilla of Vater, in the sphincter of Oddi, in the accessory pancreatic duct, or/and in the minor duodenal papilla.

3. The pharmaceutical composition for use of any one of the claims 1 to 2, wherein the obstruction is caused by a concrement located in the pancreatic duct system, or/and by lithiasis.

4. The pharmaceutical composition for use of any one of the claims 1 to 2, wherein said disease or condition is cancer.

5. The pharmaceutical composition for use of claim 4, wherein the cancer is cancer in the major pancreatic duct, in the ductus hepatopancreaticus, in the papilla of Vater, in the sphincter of Oddi, in the accessory pancreatic duct, or/and in the minor duodenal papilla.

6. The pharmaceutical composition for use of claim 4 or 5, wherein the cancer includes a tumor.

7. The pharmaceutical composition for use of any one of the preceding claims, wherein the stenosis or/and obstruction causes pain.

8. The pharmaceutical composition for use of any one of the preceding claims, wherein the treatment is a palliative treatment.

9. The pharmaceutical composition for use of any one of the preceding claims, wherein the treatment is a treatment of at least one month, at least two months, at least three months, at least four months, or at least six months.

**10.** A pharmaceutical composition for use in a palliative treatment of pain caused by a stenosis or/and obstruction in the pancreatic duct system, said composition comprising lixisenatide or/and a pharmaceutically acceptable salt thereof and optionally a pharmaceutically acceptable carrier, diluent or/and auxiliary substance.

**11.** A pharmaceutical composition for use in the treatment of pain caused by a stenosis or/and obstruction in the pancreatic duct system, said composition comprising lixisenatide or/and a pharmaceutically acceptable salt thereof and optionally a pharmaceutically acceptable carrier, diluent or/and auxiliary substance.

**Patentansprüche**

**1.** Pharmazeutische Zusammensetzung zur Verwendung bei der Behandlung einer Krankheit oder eines Zustandes, wobei die Krankheit oder der Zustand mit einer Stenose oder/und einem Verschluss einhergeht, welche/welcher im Gangsystem der Bauchspeicheldrüse und/oder im umschließenden Bauchspeicheldrüsengewebe auftritt, wobei die Zusammensetzung Lixisenatid oder/und ein pharmazeutisch akzeptables Salz desselben sowie gegebenenfalls einen pharmazeutisch akzeptables Trägerstoff, Verdünnungsstoff oder/und Hilfsstoff umfasst.

**2.** Pharmazeutische Zusammensetzung zur Verwendung nach Anspruch 1, wobei die Stenose oder/und der Verschluss im Hauptgang der Bauchspeicheldrüse, im Ductus hepatopancreaticus, in der Vaterschen Papille, im Oddischen Schließmuskel, im Nebengang der Bauchspeicheldrüse oder/und in der kleinen Zwölffingerdarmpapille auftritt.

**3.** Pharmazeutische Zusammensetzung zur Verwendung nach einem beliebigen der Ansprüche 1 bis 2, wobei der Verschluss durch ein Konkrement, das sich im Gangsystem der Bauchspeicheldrüse befindet, oder/und durch Steinbildung verursacht wird.

**4.** Pharmazeutische Zusammensetzung zur Verwendung nach einem beliebigen der Ansprüche 1 bis 2, wobei es sich bei der Krankheit oder dem Zustand um eine Krebserkrankung handelt.

**5.** Pharmazeutische Zusammensetzung zur Verwendung nach Anspruch 4, wobei es sich bei der Krebserkrankung um eine Krebserkrankung im Hauptgang der Bauchspeicheldrüse, im Ductus hepatopancreaticus, in der Vaterschen Papille, im Oddischen Schließmuskel, im Nebengang der Bauchspeicheldrüse oder/und in der kleinen Zwölffingerdarmpapille handelt.

**6.** Pharmazeutische Zusammensetzung zur Verwendung nach Anspruch 4 oder 5, wobei die Krebserkrankung einen Tumor umfasst.

**7.** Pharmazeutische Zusammensetzung zur Verwendung nach einem beliebigen der vorhergehenden Ansprüche, wobei die Stenose oder/und der Verschluss Schmerzen verursachen.

**8.** Pharmazeutische Zusammensetzung zur Verwendung nach einem beliebigen der vorhergehenden Ansprüche, wobei die Behandlung eine palliative Behandlung ist.

**9.** Pharmazeutische Zusammensetzung zur Verwendung nach einem beliebigen der vorhergehenden Ansprüche, wobei die Behandlung eine Behandlung ist, die mindestens einen Monat, mindestens zwei Monate, mindestens drei Monate, mindestens vier Monate oder mindestens sechs Monate dauert.

**10.** Pharmazeutische Zusammensetzung zur Verwendung bei einer palliativen Behandlung von Schmerzen, die von einer Stenose oder/und einem Verschluss im Gangsystem der Bauchspeicheldrüse verursacht werden, wobei die Zusammensetzung Lixisenatid oder/und ein pharmazeutisch akzeptables Salz desselben sowie gegebenenfalls einen pharmazeutisch akzeptables Trägerstoff, Verdünnungsstoff und/oder Hilfsstoff umfasst.

**11.** Pharmazeutische Zusammensetzung zur Verwendung bei der Behandlung von Schmerzen, die von einer Stenose oder/und einem Verschluss im Gangsystem der Bauchspeicheldrüse verursacht werden, wobei die Zusammensetzung Lixisenatid oder/und ein pharmazeutisch akzeptables Salz desselben sowie gegebenenfalls einen pharmazeutisch akzeptablen Trägerstoff, Verdünnungsstoff und/oder Hilfsstoff umfasst.

**Revendications**

1. Composition pharmaceutique destinée à être utilisée dans le traitement d'une maladie ou d'un état de santé, ladite maladie ou ledit état de santé étant associé(e) à une sténose et/ou à une obstruction située dans le système du canal pancréatique et/ou dans le tissu pancréatique environnant, ladite composition comprenant du lixisénatide et/ou un sel pharmaceutiquement acceptable correspondant et éventuellement un support, un diluant et/ou un adjuvant pharmaceutiquement acceptable(s).

2. Composition pharmaceutique destinée à être utilisée selon la revendication 1, la sténose et/ou l'obstruction étant située dans le canal pancréatique majeur, dans le canal hépato-pancréatique, dans l'ampoule de Vater, dans le sphincter d'Oddi, dans le canal pancréatique accessoire et/ou dans l'ampoule duodénale mineure.

3. Composition pharmaceutique destinée à être utilisée selon l'une quelconque des revendications 1 à 2, l'obstruction étant provoquée par un dépôt situé dans le système du canal pancréatique et/ou par lithiase.

4. Composition pharmaceutique destinée à être utilisée selon l'une quelconque des revendications 1 à 2, ladite maladie ou ledit état étant un cancer.

5. Composition pharmaceutique destinée à être utilisée selon la revendication 4, le cancer étant un cancer dans le canal pancréatique majeur, dans le canal hépato-pancréatique, dans l'ampoule de Vater, dans le sphincter d'Oddi, dans le canal pancréatique accessoire et/ou dans l'ampoule duodénale mineure.

6. Composition pharmaceutique destinée à être utilisée selon l'une quelconque des revendications 4 ou 5, ledit cancer incluant une tumeur.

7. Composition pharmaceutique destinée à être utilisée selon l'une quelconque des revendications précédentes, la sténose et/ou l'obstruction provoquant une douleur.

8. Composition pharmaceutique destinée à être utilisée selon l'une quelconque des revendications précédentes, le traitement étant un traitement palliatif.

9. Composition pharmaceutique destinée à être utilisée selon l'une quelconque des revendications précédentes, le traitement étant un traitement d'au moins un mois, d'au moins deux mois, d'au moins trois mois, d'au moins quatre mois ou d'au moins six mois.

10. Composition pharmaceutique destinée à être utilisée dans un traitement palliatif de la douleur provoquée par une sténose et/ou une obstruction située dans le système du tractus pancréatique, ladite composition comprenant du lixisénatide et/ou un sel pharmaceutiquement acceptable correspondant et éventuellement un support, un diluant et/ou un adjuvant pharmaceutiquement acceptable(s).

11. Composition pharmaceutique destinée à être utilisée dans un traitement de la douleur provoquée par une sténose et/ou une obstruction située dans le système du tractus pancréatique, ladite composition comprenant du lixisénatide et/ou un sel pharmaceutiquement acceptable correspondant et éventuellement un support, un diluant et/ou un adjuvant pharmaceutiquement acceptable(s).

# Figure 1

EP 2 763 690 B1

## Figure 2

Placebo

Lixisenatide

Subcutaneous
placebo injection
followed by iv bolus
of 99mTc

After GB
visualization at 60
min, start of CCK-8
infusion (0.02 µg/kg
over 60 min)

Subcutaneous
injection of 20 µg
lixisenatide
followed by iv bolus
of 99mTc

After GB
visualization at 60
min, start of CCK-8
infusion (0.02 µg/kg
over 60 min)

EP 2 763 690 B1

## Figure 3

### Placebo

Primary endpoint GBEF
(%) at 60 min

### Lixisenatide

Primary endpoint GBEF
(%) at 60 min

# Figure 4

## Mean profile plot

Treatment :　△△△ Placebo　　　⊟⊟⊟ Lixisenatide 20 ug

## Median profile plot

Treatment :　△△△ Placebo　　　⊟⊟⊟ Lixisenatide 20 ug

## Figure 5

Source = PKS Study : PDY11431; Scenario: P-D-A-EV-OD, Version 1

## Figure 6

Source = PKS Study : PDY11431; Scenario: P-D-A-EV-OD, Version 1

Figure 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5424286 A
- WO 9805351 A
- WO 9907404 A
- WO 9925727 A
- WO 9925728 A
- WO 2007028394 A
- WO 9808871 A
- WO 0104156 A
- WO 9830231 A
- EP 99610043 A
- WO 2004005342 A
- WO 04035623 A
- EP 11183867 A

### Non-patent literature cited in the description

- **KAMISAWA et al.** *J. Anat.,* 2008, vol. 212 (2), 125-34
- Gastrointestinal disease: pathophysiology, diagnosis, management. Saunders, 1973
- **NAUCK et al.** *Exp Clin Endocrinol Diabetes,* 1997, vol. 105, 187
- **LOPEZ-DELGADO et al.** *Endocrinology,* 1998, vol. 139, 2811
- **HEINRICH et al.** *Endocrinol,* 1984, vol. 115, 2176
- **UTTENTHAL et al.** *J Clin Endocrinol Metabol,* 1985, vol. 61, 472
- **GOKE et al.** *J. Biol Chem,* vol. 268, 19650-55
- **RAUFMAN.** *Reg. Peptides,* 1996, vol. 61, 1-18
- **BEHAR J. et al.** *Gastroenterology,* 2006, vol. 130, 1498-1509
- *J Gastrointestin Liver Dis.,* June 2007, vol. 16 (2), 157-61
- *Nucl Med Common.,* October 1993, vol. 14 (10), 868-72
- *Dig Liver Dis.,* July 2003, vol. 35 (3), 62-6
- **KRISHNAMURTHY GT ; KRISHNAMURTHY S ; BROWN PH.** Constancy and variability of gallbladder ejection fraction: impact on diagnosis and therapy. *J Nucl Med.,* November 2004, vol. 45 (11), 1872-7
- **ZIESSMAN HA ; TULCHINSKY M ; LAVELY WC ; GAUGHAN JP ; ALLEN TW ; MARU A et al.** Sincalide-stimulated cholescintigraphy: a multicenter investigation to determine optimal infusion methodology and gallbladder ejection fraction normal values. *J Nucl Med.,* February 2010, vol. 51 (2), 277-81
- **BURDICK R ; GRAYBILL F.** Confidence intervals on variance components. Marcel Dekker, 1992

SEQUENCE LISTING

<110> Sanofi-Aventis Deutschland GmbH

<120> GLP-1 agonist for use in the treatment of stenosis or/and obstruction in the pancreatic duct system

<130> 51174PEP

<140> EP11183867.8
<141> 2011-10-04

<160> 2

<170> PatentIn version 3.5

<210> 1
<211> 44
<212> PRT
<213> Artificial Sequence

<220>
<223> AVE0010

<220>
<221> MOD_RES
<222> (44) .. (44)
<223> AMIDATION

<400> 1

```
His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Ser Lys Lys Lys Lys Lys Lys
            35                  40
```

```
<210> 2
<211> 39
<212> PRT
<213> Artificial Sequence

<220>
<223> Exendin-4

<220>
<221> MOD_RES
<222> (39) .. (39)
<223> AMIDATION

<400> 2
```

His Gly Glu Gly Thr Phe Thr Ser Asp Leu Ser Lys Gln Met Glu Glu
1                   5                   10                  15

Glu Ala Val Arg Leu Phe Ile Glu Trp Leu Lys Asn Gly Gly Pro Ser
                20                  25                  30

Ser Gly Ala Pro Pro Pro Ser
                35